# EUROPEAN PATENT APPLICATION

(11) **EP 2 484 378 A1**
(43) Date of publication of application: **08.08.2012**
(21) Application number: 10820650.9
(22) Date of filing: 30.09.2010
(51) Int. Cl.: A61K 39/29, A61K 9/16, A61K 39/39, A61K 47/02, A61K 47/34, A61P 1/16, A61P 31/12, A61P 37/04, C07K 14/18, C07K 19/00, C12N 7/04, C12N 15/09

(54) **HEPATITIS C VIRUS VACCINE COMPOSITION**

(30) Priority: 30.09.2009 JP 2009228145
(71) Applicant: Toray Industries, Inc., Tokyo 103-8666 (JP); Japan As Represented By Director-General Of National Institute Of Infectious Diseases, Tokyo 162-8640 (JP); Tokyo Metropolitan Institute of Medical Science, Tokyo 156-0057 (JP)
(72) Inventor: WAKITA, Takaji, Tokyo 107-0062 (JP); MORIYAMA, Masaki, Kamakura-shi Kanagawa 248-8555 (JP); AKAZAWA, Daisuke, Kamakura-shi Kanagawa 248-8555 (JP); NAKAMURA, Noriko, Kamakura-shi Kanagawa 248-8555 (JP)
(74) Representative: Kador & Partner
(86) International application number: PCT/JP2010/067096
(87) International publication number: WO 2011/040535

(57) **Abstract**

A combination of an HCV antigen for inducing an antibody having the activity of inhibiting HCV infection and an optimum adjuvant is discovered, so as to provide an effective HCV vaccine composition. The hepatitis C virus vaccine composition comprises: inactivated viral particles obtained by inactivating infectious hepatitis C virus particles prepared from hepatitis C virus genome that contains sequences encoding NS3 protein, NS4A protein, NS4B protein, NS5A protein and NS5B protein derived from hepatitis C virus JFH1 strain;
an oligonucleotide containing unmethylated CpG, according to SEQ ID NO: 5 in the sequence listing; and
aluminium hydroxide.

## Description

### Technical Field

The present invention relates to a hepatitis C virus vaccine composition.

### Background Art

The hepatitis C virus (which may be abbreviated as "HCV" hereinafter) was discovered as a major causative virus of non-A and non-B hepatitis (non-patent document 1). HCV is a single-stranded (+) RNA virus having a genome length of approximately 9.6 kb, in which the genome encodes a precursor protein that is divided into 10 types of virus protein (i.e., Core, E1, E2, p7, NS2, NS3, NS4A, NS4B, NS5A, and NS5B proteins) via post-translational cleavage by signal peptidase from host or proteases from HCV. Of these virus proteins, Core, E1, E2, and p7 proteins are classified as structural proteins, and NS2, NS3, NS4A, NS4B, NS5A, and NS5B proteins are classified as non-structural proteins. HCV is also classified into 10 or more genotypes (e.g., 1a, 1b, 2a, 2b, 3a, and 3b) depending on differences in the nucleotide sequences of the genomes (Non-patent documents 2 to 4). Genotypes can be determined by an HCV antibody test, an HCV core antigen test, or a nucleic acid amplification test.

HCV is transmitted from human to human via blood, causing chronic hepatitis among about 60% to 80% of infected persons. When hepatitis is left without any appropriate treatment, it is known to cause cirrhosis or liver cancer within about 20 to 30 years after infection. Therefore, early detection of HCV infection for prevention of the onset of chronic hepatitis and early treatment of the disease after the onset thereof are desired.

Generally, a major therapeutic method for hepatitis C entails administration of interferon alone or combination therapy with interferon and ribavirin. However, it has been recently revealed that the therapeutic effects of interferons differ significantly depending on differences in HCV genotypes, and the effects are not easily exhibited on HCV of genotype 1a or 1b (non-patent documents 5 and 6). It has also been revealed that the intensity of the antiviral action of interferons varies even between HCV of genotype 2a and HCV of genotype 2b, upon which the relatively good effects of interferon are observed. It has been suggested that interferons exhibit antiviral action more strongly against HCV of genotype 2a than against HCV of genotype 2b (non-patent document 7).

Hence, the development of an HCV vaccine that makes it possible to prevent the onset of chronic hepatitis in virus carriers who have not yet developed chronic hepatitis after infection with HCV or to enhance autoimmunity after the onset of chronic hepatitis, so as to eliminate HCV, is desired.

However, both obtainment of HCV particles with infectivity (hereinafter, may be abbreviated as "infectious HCV particles") and testing of the effects of inhibiting HCV infection are difficult. Moreover, it is difficult to find a combination of an adjuvant and an antigen that enhances humoral immunity and cellular immunity required for exhibition of the effects of inhibiting HCV infection. Hence, no HCV vaccine composition having sufficient activity of inhibiting HCV infection has yet been developed.

In general, adjuvants can be classified based on physical properties into particulate adjuvants and non-particulate adjuvants.

Examples of a particulate adjuvant include aluminum salts, water-in-oil type emulsions, oil-in-water type emulsions, immune-stimulating complexes, liposomes, nanoparticles, and fine particles. A particulate adjuvant alone is often mixed with an antigen and then used. In particular, aluminium hydroxide (hereinafter, may be abbreviated as "Alum") is used for medicinal use as a low inflammatory adjuvant. However, it is known that a combination of Alum and an antigen having medium to low antigen titer is unable to enhance humoral immunity and cellular immunity as required for exhibition of the effects of inhibiting infection. Thus, Alum is inappropriate for use as an adjuvant.

Meanwhile, examples of a non-particulate adjuvant include a muramyl dipeptide (an active ingredient of an adjuvant of peptide glycan extracted from Mycobacteria), a nonionic block copolymer, saponin (a complex mixture of triterpenoid extracted from *Quillaja saponaria* bark), lipid A (glycosamine disaccharides with five or six C12-C16 fatty acid chains and 2 phosphate radicals), cytokine, nucleic acid, a carbohydrate polymer, derivatized polysaccharides, and bacterial toxin (e.g., cholera toxin and *E. coli* thermolabile toxin), which are often used in combination with a particulate adjuvant.

As nucleic acid to be used as an adjuvant, oligodeoxynucleotide containing a CpG dinucleotide motif that has not undergone methylation modification (hereinafter, may be abbreviated as "CpG-ODN") (non-patent document 8) and double-stranded RNA (hereinafter, may be abbreviated as "dsRNA") are known.

It has been reported that CpG-ODN containing a CpG motif consisting of a 6-base palindrome motif induces strong cellular cytotoxicity. In particular, 3 sequences (5'-AACGTT-3', 5'-AGCGCT-3', and 5'-GACGTC-3') particularly strongly induce cellular cytotoxicity (non-patent document 9). It has recently been revealed that CpG-ODN is a ligand of TLR9 and induces immune response through activation of TLR9.

Also, a typical example of dsRNA is polyriboinosinic acid-polyribocytidylic acid (hereinafter, referred to as "polyI:C") prepared by hybridization of polyriboinosinic acid (polyI) with polyribocytidylic acid (polyC). Furthermore, polyICLC, which is a complex of polyI:C, poly L lysine, and carboxymethyl cellulose (non-patent document 10), and polyI:PolyC12U, in which the C portion in polyI:C has been substituted with U (specifically, one of every 12 Cs has been substituted with "U"), are known as less toxic dsRNAs (non-patent document 11). It has been revealed that polyI:C is an inducer of interferon I and a ligand of TLR3.

Also, recombinant proteins (patent documents 1 and 2), synthetic peptides (patent document 3), virus-like particles (patent document 4), naked DNA (patent document 5), and viral particles (patent documents 6 and 7) have been reported as antigen protein candidates to be used for development of an HCV vaccine.

Patent document 1 discloses that antibody titers against the E1 and E2 proteins are increased by mixing the E1 and E2 proteins, which are HCV structural proteins, as antigens with MF59 (submicronic oil-in-water type emulsion) and CpG-ODN as adjuvants, but does not reveal the thus induced antibody's activity of inhibiting HCV infection.

Furthermore, patent document 2 discloses that the production amounts of antibodies against the E1 and E2 proteins are increased by mixing the HCV E1 and E2 proteins as antigens with polyI:C as an adjuvant, but similarly to patent document 1, it does not demonstrate the antibodies' activity of inhibiting HCV infection.

Furthermore, patent documents 6 and 7 describe a vaccine using an HCV particle itself as an antigen, but do not describe any adjuvant, or else merely describe general examples thereof. Moreover, patent documents 6 and 7 never mention the antibody production capacity of the vaccine or the antibody's activity of inhibiting HCV infection.

Furthermore, non-patent document 12 discloses that the production amounts of antibodies against Core, NS3, NS4, and NS5 proteins are increased by mixing HCV Core, NS3, NS4, and NS5 proteins as antigens with Alum and CpG-ODN as adjuvants. However, similarly to patent documents 1 and 2, the document does not disclose the antibodies' activity of inhibiting HCV infection.

### Prior Art Documents

### Patent documents

Patent document 1 JP Patent Publication (Kohyo) No. 2005-502611 A
Patent document 2 JP Patent No. 4286138
Patent document 3 JP Patent Publication (Kohyo) No. 2009-513542 A
Patent document 4 JP Patent Publication (Kohyo) No. 2001-504337 A
Patent document 5 JP Patent Publication (Kokai) No. 2009-183295 A
Patent document 6 International Patent Publication No. 05/080575
Patent document 7 International Patent Publication No. 06/022422

### Non-patent documents

Non-patent document 1 Choo et al., Science, 1989, Vol. 244, p. 359-362
Non-patent document 2 Simmonds et al., Hepatology, 1994, Vol. 10, p. 1321-1324
Non-patent document 3 Okamoto et al., J. Gen. Virol., 1992, Vol. 73, p. 73-679
Non-patent document 4 Mori et al., Biochem. Biophys. Res. Commun., 1992, Vol. 183, p. 334-342
Non-patent document 5 Fried et al., N. Engl. J. Med., 2002, Vol. 347, p. 975-982
Non-patent document 6 Lusida et al., J. Clin. Microbiol., 2001, Vol. 39, p.3858-3864
Non-patent document 7 Murakami et al., Hepatology, 1999, Vol. 30, p. 1045-1053
Non-patent document 8 Yamamoto et al., Jpn, J. Cancer Res. 1988, Vol. 79, p. 866-873
Non-patent document 9 Yamamoto et al., J. Immunol. 1992, Vol. 148, p. 4072-4076
Non-patent document 10 Nakamura et al., J. Interferon Res. 1982, Vol. 2, p. 1-4
Non-patent document 11 Laurence et al., J. Clin. Invest. 1987, Vol. 80, p. 1631-1639
Non-patent document 12 Vajdy et al., J. Gen. Virol. 2006, Vol. 87, p. 2253-2262

### Summary of the Invention

### Problem to be Solved by the Invention

An object of the present invention is to provide an effective HCV vaccine composition through discovery of a combination of an HCV antigen for inducing an antibody having activity of inhibiting HCV infection and an optimum adjuvant therefor.

### Means for Solving the Problem

A mouse was immunized with the E2 protein, which is an envelope protein required for HCV particles to adhere to and infect cells, as an antigen, and then antibody titer against the E2 protein in the serum was evaluated. High antibody titer was observed. It has conventionally been believed that antibody titer against the E2 protein correlates with the antibody's activity of inhibiting HCV infection. However, as a result of the study by the present inventors, it has been surprisingly revealed that this hypothesis is not always true. Specifically, as described in the examples of the description, the present inventors demonstrated that when a mouse was inoculated with a vaccine containing Alum and CpG-ODN as adjuvants and the E2 protein, antibody titer against the E2 protein in serum was increased, but the activity of inhibiting HCV infection was not increased.

Hence, to search for a combination of an HCV antigen for inducing an antibody having activity of inhibiting HCV infection and an adjuvant, infectious HCV particles were produced in a cell culture system, purified, and then inactivated, and then vaccine compositions combined with various adjuvants were prepared and then administered to mice. Antibody titer against the HCV E2 protein in serum of each mouse after administration and activity of inhibiting HCV infection were measured. Thus, it was found that the activity of inhibiting HCV infection does not always reach a high level, even if an adjuvant exhibiting high-level antibody titer against the HCV E2 protein is used as described above, but the use of a vaccine composition consisting of inactivated HCV particles + Alum + CpG-ODN results in not only high-level antibody titers against the E1 protein and the E2 protein, but also high-level activity of inhibiting HCV infection. Thus, the present invention was completed. Specifically, the present invention encompasses the following.

The present invention provides a hepatitis C virus vaccine composition, comprising:
inactivated viral particles obtained by inactivating infectious hepatitis C virus particles prepared from the hepatitis C virus genome that contains sequences encoding NS3 protein, NS4A protein, NS4B protein, NS5A protein and NS5B protein derived from the hepatitis C virus JFH1 strain;
an oligonucleotide containing unmethylated CpG shown in SEQ ID NO: 5 in the sequence listing; and
aluminium hydroxide. The infectious hepatitis C virus genome is preferably the hepatitis C virus vaccine composition containing sequences that encode core protein, E1 protein, E2 protein and p7 protein derived from the hepatitis C virus J6CF strain.

The infectious hepatitis C virus genome is also preferably the above hepatitis C virus vaccine composition containing a sequence that encodes 16 amino acid residues from the N-terminal acid residue of NS2 protein derived from the hepatitis C virus J6CF strain and a sequence that encodes a portion ranging from the 17^{th} amino acid residue from the N terminus to the C-terminal amino acid residue of NS2 protein derived from the hepatitis C virus JFH1 strain.

Furthermore, the present invention provides a hepatitis C virus vaccine composition containing:
inactivated viral particles obtained by inactivating infectious hepatitis C virus particles containing NS3 protein, NS4A protein, NS4B protein, NS5A protein and NS5B protein derived from the hepatitis C virus JFH1 strain;
an oligonucleotide containing the unmethylated CpG shown in SEQ ID NO: 5 in the sequence listing; and
aluminium hydroxide. The infectious hepatitis C virus is preferably the above hepatitis C virus vaccine composition containing core protein, E1 protein, E2 protein and p7 protein derived from the hepatitis C virus J6CF strain.

The infectious hepatitis C virus is also preferably the above hepatitis C virus vaccine composition in which the NS2 protein is a chimeric protein, 16 amino acid residues from the N-terminal amino acid residue of the NS2 protein are derived from the J6CF strain, and a portion ranging from the 17th amino acid residue from the N terminus to the C terminal amino acid residue of the NS2 protein is derived from the JFH1 strain.

Furthermore, the present invention provides a hepatitis C virus vaccine composition containing:
inactivated hepatitis C virus particles of genotype 2a;
an oligonucleotide (CpG-ODN) containing the unmethylated CpG shown in SEQ ID NO: 5 in the sequence listing; and
aluminium hydroxide.
The hepatitis C virus particles of genotype 2a preferably have structural proteins of hepatitis C virus of genotype 2a, and particularly structural proteins (core protein, E1 protein, E2 protein, and p7 protein) derived from the J6CF strain. The hepatitis C virus particles of genotype 2a preferably have the E2 protein of hepatitis C virus of genotype 2a and particularly E2 protein derived from the J6CF strain. Furthermore, the hepatitis C virus particles of genotype 2a are preferably viral particles produced by expression of the HCV genome having nucleotide sequences that encode the NS2 protein derived from at least 1 type of hepatitis C virus strain, NS3 protein, NS4A protein, NS4B protein, NS5A protein and NS5B protein derived from the JFH1 strain. The hepatitis C virus particles of genotype 2a are also preferably viral particles produced by expression of a chimeric HCV genome containing nucleotide sequences that encode structural proteins (core protein, E1 protein, E2 protein, and p7 protein) derived from J6CF strain, the NS2 protein that is a chimeric protein derived from the J6CF strain and JFH1 strain (16 amino acid residues from the N-terminal amino acid residue of the NS2 protein are derived from the J6CF strain and a portion ranging from the 17^{th} amino acid residue from the N terminus to the C-terminal amino acid residue of the NS2 protein is derived from the JFH1 strain), and non-structural proteins other than NS2 derived from the JFH1 strain (NS3 protein, NS4A protein, NS4B protein, NS5A protein, and NS5B protein), the 5' untranslated region, and the 3' untranslated region.

This description includes the disclosure of the description and/or drawings of Japanese Patent Application No. 2009-228145, from which the present application claims priority.

### Effects of the Invention

The present invention provides a vaccine composition for effectively preventing hepatitis C virus infection.

### Brief Description of the Drawings

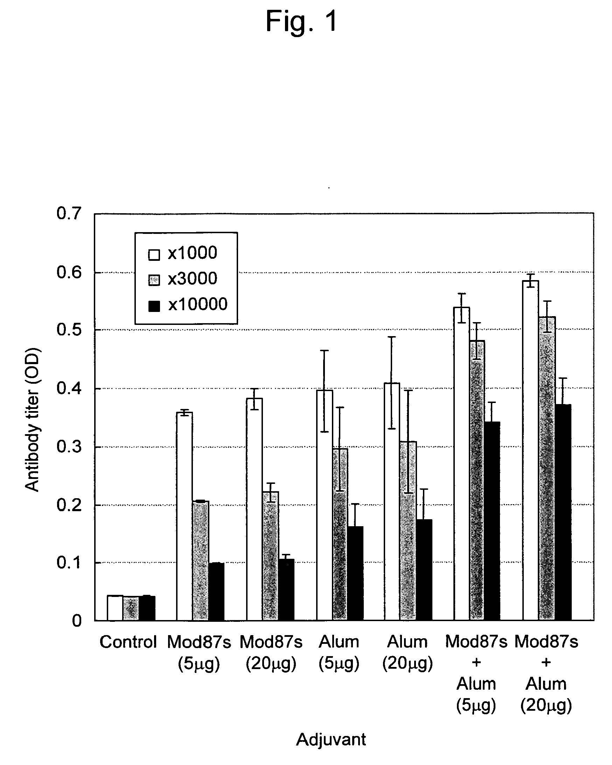

Fig. 1 shows the results of measuring antibody titer against the J6E2 protein by EIA when a J6E2Fc protein (5 µg or 20 µg) as an antigen and Alum or Mod87s alone or in combination as an adjuvant were administered twice to mice at 2 week intervals, serum samples were collected 10 days after the final administration, and then the samples were diluted 1000 fold, 3000 fold, and 10000 fold.

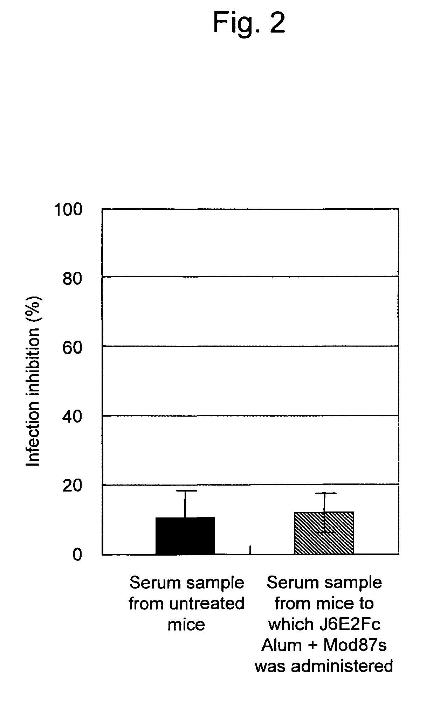

Fig. 2 shows the results of measuring the inhibition activity against J6CF HCVpp infection when a J6E2Fc protein (20 µg) as an antigen and Alum+Mod87s as an adjuvant were administered twice to mice at 2 week intervals, serum samples were collected 10 days after the final administration, and then the serum samples were diluted 100 fold.

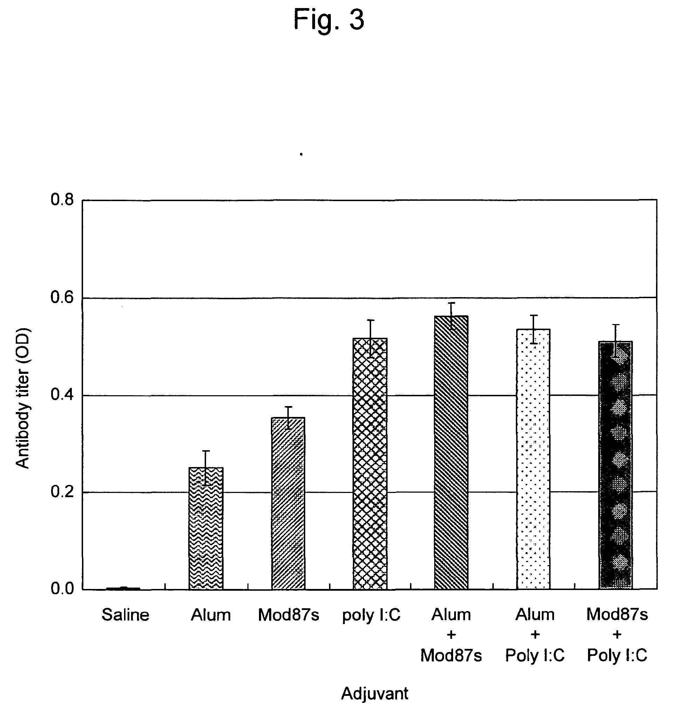

Fig. 3 shows the results of measuring antibody titers against the E1 protein when inactivated J6/JFH1-HCV particles (2 pmol) as antigens and Alum, Mod87s, or polyI:C alone or in combination as an adjuvant were administered 4 times to mice at 2 week intervals, serum samples were collected 1 week after the final administration, and then the serum samples were diluted 3000 fold. In Fig. 3, "Saline" denotes the serum of mice to which saline alone was administered.

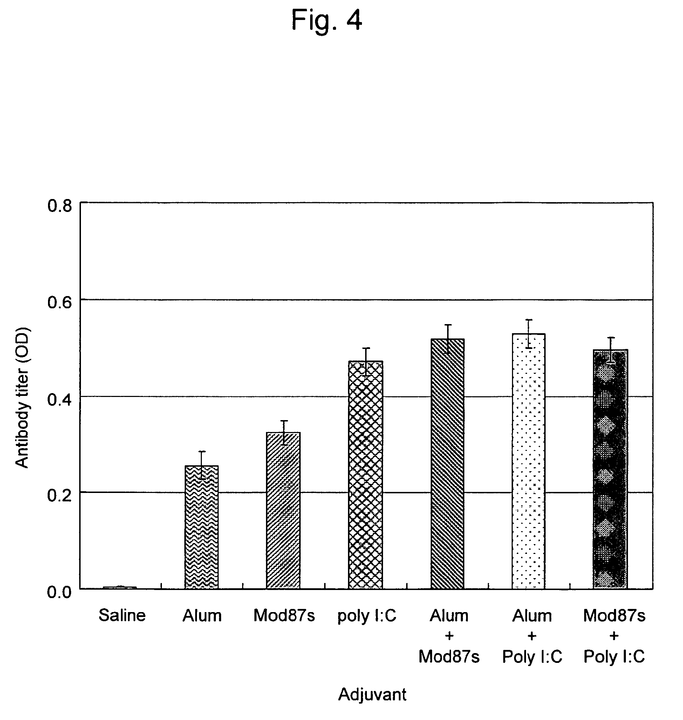

Fig. 4 shows the results of measuring antibody titers against the E2 protein when inactivated J6/JFH1-HCV particles (2 pmol) as an antigen and Alum, Mod87s or polyI:C alone or in combination as an adjuvant were administered 4 times to mice at 2 week intervals, serum samples were collected 1 week after the final administration, and then the serum samples were diluted 3000 fold. In Fig. 4, "Saline" denotes the serum of mice to which saline alone was administered.

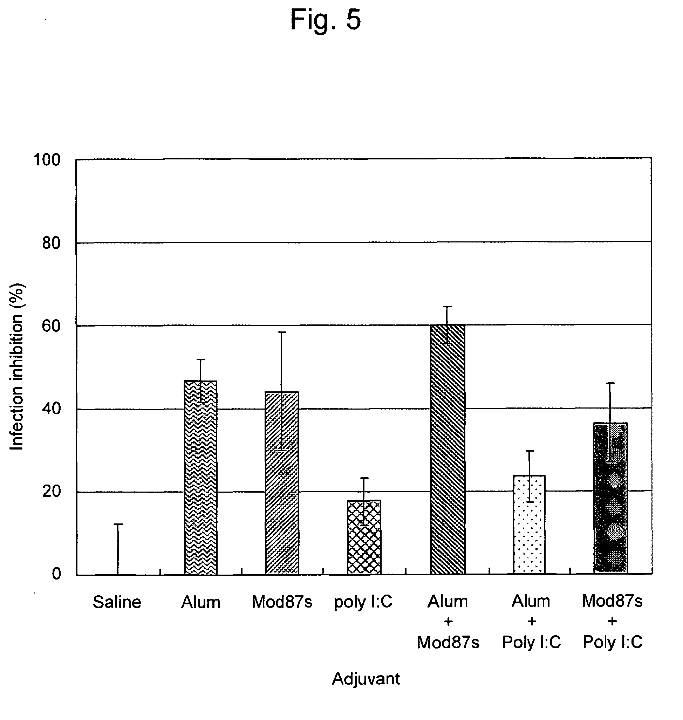

Fig. 5 shows the results of measuring the inhibition activity against J6CF HCVpp (genotype 2a) infection when inactivated J6/JFH1-HCV particles (2 pmol) as an antigen and Alum, Mod87s or polyI:C alone or in combination as an adjuvant were administered 4 times to a mouse at 2 week intervals, serum samples were collected 1 week after the final administration, and then the serum samples were diluted 100 fold. In Fig. 5, "Saline" denotes the serum of a mouse to which saline alone was administered.

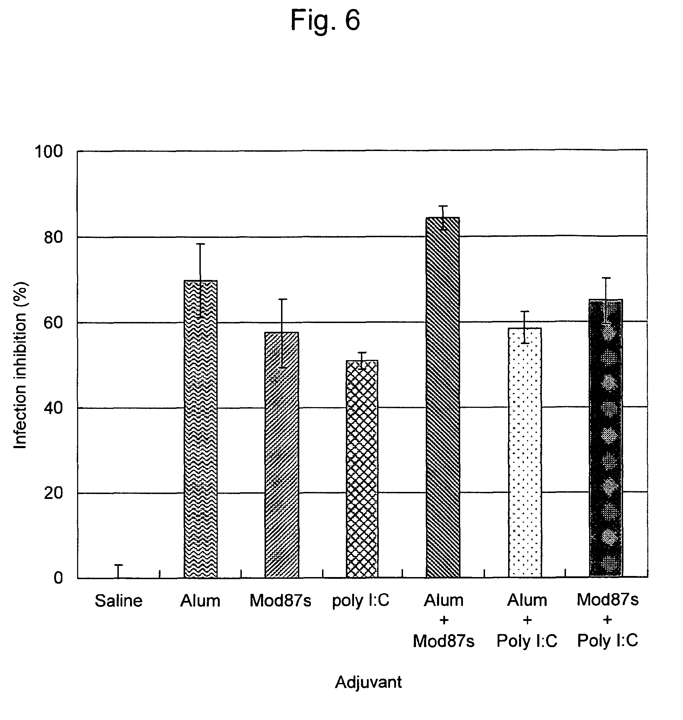

Fig. 6 shows the results of measuring the inhibition activity against H77 HCVpp (genotype 1a) infection when inactivated J6/JFH1-HCV particles (2 pmol) as an antigen and Alum, Mod87s or polyI:C alone or in combination as an adjuvant were administered 4 times to mice at 2 week intervals, serum samples were collected 1 week after the final administration, and then the serum samples were diluted 100 fold. In Fig. 6, "Saline" denotes the serum of mice to which saline alone was administered.

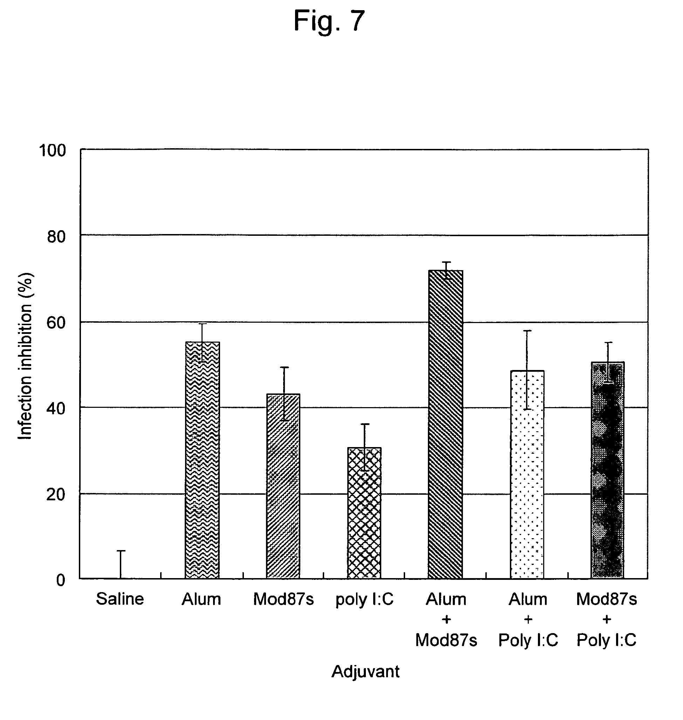

Fig. 7 shows the results of measuring the inhibition activity against TH HCVpp (genotype 1b) infection when inactivated J6/JFH1-HCV particles (2 pmol) as an antigen and Alum, Mod87s or polyI:C alone or in combination as an adjuvant were administered 4 times to mice at 2 week intervals, serum samples were collected 1 week after the final administration, and then the serum samples were diluted 100 fold. In Fig. 7, "Saline" denotes the serum of mice to which saline alone was administered.

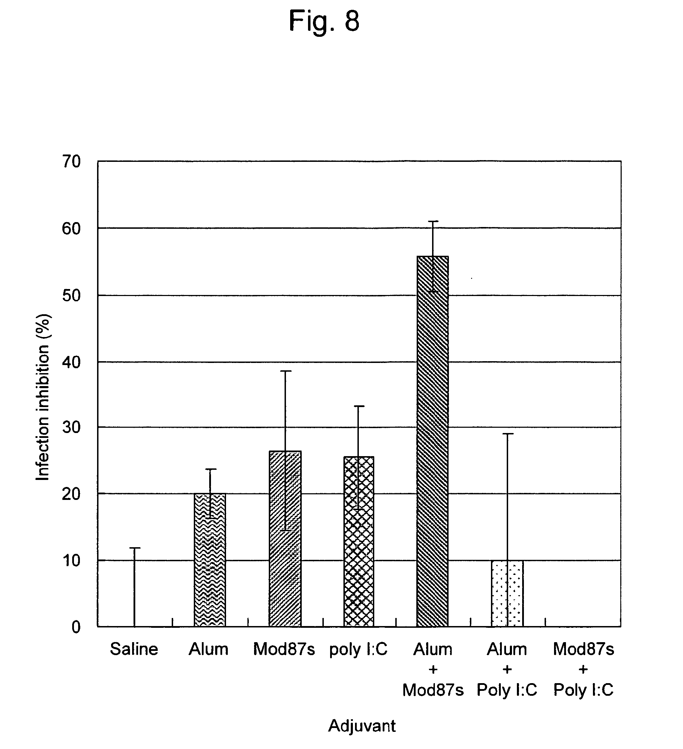

Fig. 8 shows the results of measuring the inhibition activity against J6/JFH1 HCVcc (genotype 2a) infection when inactivated J6/JFH1-HCV particles (2 pmol) as an antigen and Alum, Mod87s or polyI:C alone or in combination as an adjuvant were administered 4 times to mice at 2 week intervals, serum samples were collected 1 week after the final administration, and then the serum samples were diluted 100 fold. In Fig. 8, "Saline" denotes the serum of mice to which saline alone was administered.

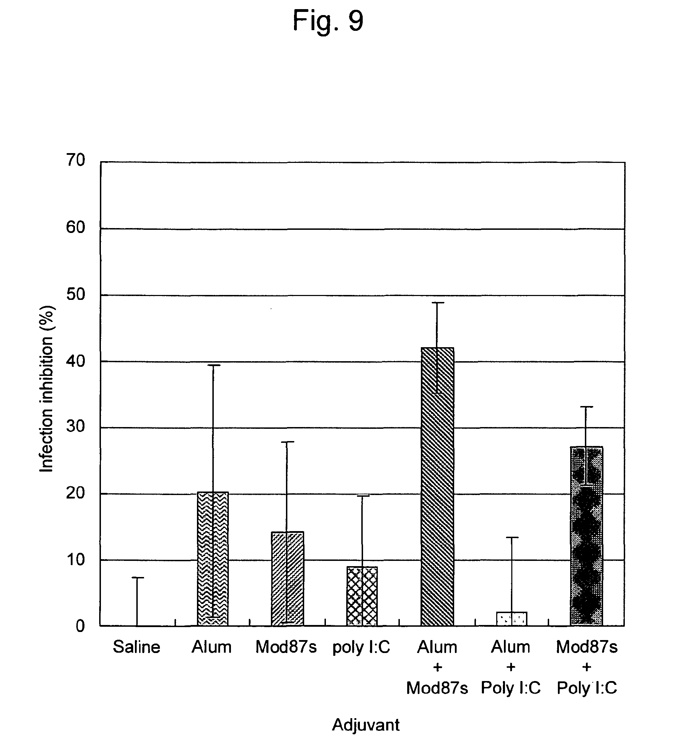

Fig. 9 shows the results of measuring the inhibition activity against TH/JFH1 HCVcc (genotype 1b) infection when inactivated J6/JFH1-HCV particles (2 pmol) as an antigen and Alum, Mod87s or polyI:C alone or in combination as an adjuvant were administered 4 times to mice at 2 week intervals, serum samples were collected 1 week after the final administration, and then the serum samples were diluted 100 fold. In Fig. 9, "Saline" denotes the serum of mice to which saline alone was administered.

### Embodiments for Carrying out the Invention

The present invention will be further described specifically as follows.

The present invention relates to a vaccine composition comprising an adjuvant and an antigen appropriate for inducing an antibody for inhibiting HCV infection, which is produced by inactivating HCV particles (preferably, infectious HCV particles) produced from a specific HCV genome and then using the HCV particle as the antigen.

The present invention can be implemented via conventional molecular biological and immunological techniques within the technical scope in the art. Such techniques are thoroughly described in, for example, Sambrook et al., Molecular Cloning: A Laboratory Manual, Cold Spring Harbor Laboratory (vol. 3, 2001) or Ed Harlow et al., Antibodies: A Laboratory Manual, Cold Spring Harbor Laboratory, 1988.

All publications, patents, and patent applications cited herein are incorporated herein by reference in their entirety.

### (1) Preparation of HCV particles

A specific example of an HCV genome that can be used for generating infectious HCV particles, which can be used as antigens for the HCV vaccine composition according to the present invention, is viral genome RNA of the JFH1 strain of genotype 2a, which consists of nucleotide sequences of, in order from the 5' side to the 3' side, a 5' untranslated region, a core protein coding region, an E1 protein coding region, an E2 protein coding region, a p7 protein coding region, an NS2 protein coding region, an NS3 protein coding region, an NS4A protein coding region, an NS4B protein coding region, an NS5A protein coding region, an NS5B protein coding region, and a 3' untranslated region. Alternatively, such an HCV genome may be chimeric RNA consisting of viral genome RNAs of 2 or more types of HCV strain. For example, such a chimeric RNA consists of the nucleotide sequences of, in order from the 5' side to the 3' side, a 5' untranslated region, a core protein coding region, an E1 protein coding region, an E2 protein coding region, and a p7 protein coding region of an HCV strain other than the JFH1 strain, an NS2 protein coding region of an HCV strain other than the JFH1 strain or the JFH1 strain, and the NS3 protein coding region, the NS4A protein coding region, the NS4B protein coding region, the NS5A protein coding region, the NS5B protein coding region, and the 3' untranslated region of the JFH1 strain. Alternatively, such an HCV genome may be chimeric RNA consisting of nucleotide sequences of, in order from the 5' side to the 3' side, a 5' untranslated region, a core protein coding region, an E1 protein coding region, an E2 protein coding region, and a p7 protein coding region of an HCV strain other than the JFH1 strain, and a chimeric NS2 protein coding region, an NS3 protein coding region, an NS4A protein coding region, an NS4B protein coding region, an NS5A protein coding region, an NS5B protein coding region, and a 3' untranslated region derived from one or more HCV strains.

Therefore, a preferred embodiment of the HCV genome to be used for preparation of infectious HCV particles that are used for the present invention contains nucleotide sequences encoding at least the NS3 protein, the NS4A protein, the NS4B protein, the NS5A protein, and the NS5B protein, respectively, which are non-structural proteins of the JFH1 strain. Such a virus that has acquired autonomous replication capacity and virus production capacity and contains the full-length or a portion of the genome of the JFH1 strain is referred to as "JFH1-derived virus."

An example of the HCV genome in a preferred embodiment of the present invention is an HCV genome consisting of, in order from the 5' side to the 3' side, a full-length genome derived from the JFH1 strain of genotype 2a (for example, a nucleic acid having the nucleotide sequence shown in SEQ ID NO: 1, provided that nucleotide "T (thymine)" in the nucleotide sequence shown in SEQ ID NO: 1 is read as "U (uracil)" when the genome is RNA. The same applies to other nucleotide sequences in the description). Alternatively, such an HCV genome is a chimeric genome consisting of nucleotide sequences of, in order from the 5' side to the 3' side, the 5' untranslated region, the core protein coding region, the E1 protein coding region, the E2 protein coding region, the p7 protein coding region, and 16 amino acid residues from the N terminal amino acid residue of the NS2 protein coding region derived from the J6CF strain of genotype 2a, and a portion of the NS2 protein coding region which ranges from the 17th amino acid residue from the N terminus to the C terminal amino acid residue, the NS3 protein coding region, the NS4A protein coding region, the NS4B protein coding region, the NS5A protein coding region, the NS5B protein coding region, and the 3' untranslated region from the JFH1 strain. A particularly preferable example thereof is a nucleic acid cloned into J6/JFH1 consisting of the nucleotide sequence of SEQ ID NO: 2.

In a particularly preferred embodiment of the present invention, the above HCV (chimera) genome is a nucleic acid consisting of the nucleotide sequence shown in SEQ ID NO: 1 or 2. HCV genome RNA or HCV genome DNA can be used for producing the infectious HCV particles of the present invention.

Furthermore, an example of the HCV genome in a preferred embodiment of the present invention is:
a chimeric HCV genome, in which the nucleotide sequences of the 5' untranslated region from the JFH1 strain, the core protein coding region, the E1 protein coding region, the E2 protein coding region, and the p7 protein coding region from the TH strain of genotype 1b (Wakita, T. et al., J. Biol. Chem., 269, 14205-14210, 1994, JP Patent Publication (Kokai) No. 2004-179 A), and the NS2 protein coding region, the NS3 protein coding region, the NS4A protein coding region, the NS4B protein coding region, the NS5A protein coding region, the NS5B protein coding region, and the 3' untranslated region from the JFH1 strain are linked in this order; or is preferably
a chimeric HCV genome, in which the 5' untranslated region derived from the JFH1 strain, the core protein coding region, the E1 protein coding region, the E2 protein coding region, the p7 protein, and a portion of the NS2 protein coding region encoding 33 amino acid residues from the N terminus from the TH strain, and a portion encoding the 34^{th} amino acid residue from the N terminus to the C terminal amino acid residue of the NS2 protein coding region, the NS3 protein coding region, the NS4A protein coding region, the NS4B protein coding region, the NS5A protein coding region, the NS5B protein coding region, and the 3' untranslated region from the JFH1 strain are linked in this order. A particularly preferable example thereof is a nucleic acid cloned into TH/JFH1 shown in the nucleotide sequence of SEQ ID NO: 3.

In a particularly preferred embodiment of the present invention, the above chimeric HCV genome is a nucleic acid consisting of the nucleotide sequence shown in SEQ ID NO: 3. HCV genome RNA or HCV genome DNA can be used for producing the infectious HCV particles of the present invention.

Specifically, the present invention relates to a vaccine for which antigens are:
HCV particles obtained from a chimeric HCV genome in which the nucleotide sequences of, in order from the 5' side to the 3' side, the 5' untranslated region, the core protein coding region, the E1 protein coding region, the E2 protein coding region, and the p7 protein coding region from the HCV J6CF strain, and the NS2 protein coding region, the NS3 protein coding region, the NS4A protein coding region, the NS4B protein coding region, the NS5A protein coding region, the NS5B protein coding region, and the 3' untranslated region from the JFH1 strain are linked; or preferably
HCV particles obtained from a chimeric HCV genome, in which the nucleotide sequences of, in order from the 5' side to the 3' side, the 5' untranslated region, the core protein coding region, the E1 protein coding region, the E2 protein coding region, the p7 protein coding region, and a sequence encoding 16 amino acid residues from the N terminal amino acid residue of the NS2 protein coding region from the J6CF strain, and a portion ranging from (encoding) the 17th amino acid residue from the N-terminus to the C terminal amino acid residue of the NS2 protein coding region, the NS3 protein coding region, the NS4A protein coding region, the NS4B protein coding region, the NS5A protein coding region, the NS5B protein coding region, and the 3' untranslated region from the JFH1 strain are linked.

Also, an example of the HCV genome in another preferred embodiment of the present invention is an HCV genome to be used for preparing infectious HCV particles that are used in the present invention, which contains at least nucleotide sequences encoding the core protein, the E1 protein, the E2 protein, and the p7 protein, respectively, that are structural proteins of the J6CF strain of genotype 2a.

The above infectious HCV particles can be prepared by synthesizing RNA from a vector in which the cDNA of the above full-length HCV genome RNA has been cloned downstream of a transcriptional promoter (e.g., a vector in which HCV genome DNA has been cloned under the control of T7 promoter), and then introducing the RNA into cells. When a method for preparing RNA *in vitro* with the use of the nucleic acid as a template, in which HCV cDNA has been cloned under the control of T7 promoter, is employed, RNA can be synthesized using a MEGAscript T7 kit (Ambion), for example. Cells into which RNA is introduced may be cells that enable the formation of HCV particles and examples thereof include cultured cells such as Huh7 cells, HepG2 cells, IMY-N9 cells, HeLa cells, and 293 cells. More preferable examples thereof include liver-derived cultured cells such as Huh7 cells. Further preferable examples thereof include Huh7 cells and cells of a derivative strain of Huh7 (e.g., Huh7.5 cells and Huh7.5.1 cells). Furthermore, examples thereof include Huh7 cells, HepG2 cells, IMY-N9 cells, HeLa cells, or 293 cells in which CD81 gene and/or Claudin1 gene has been expressed. In particular, Huh7 cells or cells of a derivative strain of Huh7 are preferably used. The term "derivative strain" in the present invention refers to a cell line induced from the relevant cells.

Any known method can be used as a method for introducing RNA into cells. Examples of such a method include calcium phosphate coprecipitation, a DEAE dextran method, lipofection, microinjection, and electroporation. Preferable examples thereof include lipofection and electroporation. A further preferable example thereof is electroporation.

The capacity of cells to produce viral particles can be detected using an antibody against an element (e.g., a core protein, an E1 protein, or an E2 protein) composing HCV viral particles that are released into a culture solution. Also, HCV genome RNA contained in HCV viral particles in a culture solution is amplified by an RT-PCR method using specific primers for detection, so that the presence of HCV viral particles can also be detected indirectly.

Whether or not the prepared viral particles are infectious can be evaluated by treating HCV infection-susceptible cells (e.g., Huh7 cells) with the supernatant obtained by culturing cells into which HCV RNA has been introduced in the aforementioned manner, followed by, for example, after 48 hours, immunologically staining the cells with an anti-core antibody to count the number of infected cells. Alternatively, evaluation can be carried out by subjecting the cell extract to electrophoresis on SDS-polyacrylamide gel and detecting core proteins via Western blotting.

In the description, viral particles prepared from a full-length genome derived from the JFH1 strain are referred to as "JFH1-HCV", viral particles prepared from a J6/JFH1 genome are referred to as "J6/JFH1-HCV," and viral particles prepared from a TH/JFH1 genome referred to as "TH/JFH1-HCV." These viral particles are viruses derived from JFH1.

In the present invention, hepatitis C virus (HCV) particles of genotype 2a are purified as necessary, as described below, and then inactivated, so that the resultant can be used as an antigen for the vaccine composition according to the present invention. Preferable hepatitis C virus (HCV) particles of genotype 2a have structural proteins of hepatitis C virus of genotype 2a, particularly, the structural proteins (the core protein, the E1 protein, the E2 protein, and the p7 protein) derived from J6CF strain. The hepatitis C virus (HCV) particles of genotype 2a preferably have the E2 protein of hepatitis C virus of genotype 2a and particularly, the J6CF-strain-derived E2 protein. A preferable example of the polyprotein region consisting of the structural proteins (the core protein, the E1 protein, the E2 protein, and the p7 protein) derived from J6CF strain consists of an amino acid sequence encoded by the nucleotide sequence ranging from the 341^{st} to 2779^{th} amino acid residues on the sequence shown in SEQ ID NO: 2.

Furthermore, the hepatitis C virus (HCV) particles of genotype 2a are preferably viral particles that are produced by expression of an HCV genome containing nucleotide sequences encoding an NS2 protein derived from at least 1 type of hepatitis C virus strain, and NS3 protein, NS4A protein, NS4B protein, NS5A protein, and NS5B protein derived from the JFH1 strain for the reason that they have infectivity and autonomous replication capacity. Such a hepatitis C virus (particles) of genotype 2a may be a chimeric virus (particles). The hepatitis C virus (HCV) particles of genotype 2a are preferably viral particles produced by the expression of a chimeric HCV genome that comprises: nucleotide sequences encoding a 5' untranslated region derived from any one of hepatitis C virus strains, the structural proteins (the core protein, the E1 protein, the E2 protein, and the p7 protein) derived from J6CF strain, an NS2 protein which is a chimeric protein derived from the J6CF strain and JFH1 strain (a portion ranging from the N-terminal amino acid residue to the 16^{th} amino acid residue of the NS2 protein is derived from the J6CF strain, and a portion ranging from the 17^{th} amino acid residue from the N terminal acid residue to the C-terminal amino acid residue of the NS2 protein is derived from the JFH1 strain), and non-structural proteins (NS3 protein, NS4A protein, NS4B protein, NS5A protein, and NS5B protein) derived from the JFH1 strain other than NS2; and a nucleotide sequence containing a 3' untranslated region derived from any one of hepatitis C virus strains.

### (2) Purification of viral particles

The virus solution containing the HCV particles obtained in (1) above is subjected to, for example, centrifugation and/or filtration through a filter to remove cells and cell residues. A solution from which residues have been removed can also be concentrated approximately 10- to 100-fold using an ultrafiltration membrane with a molecular weight cut off of 100,000 to 500,000. A solution containing HCV particles from which residues have been removed can be purified via chromatography and density-gradient centrifugation (described later) in arbitrary combinations in any order or alone. Hereafter, representative chromatography or density-gradient centrifugation techniques are described, although the techniques are not limited thereto.

In gel filtration chromatography, a chromatography support comprising a cross-linked polymer of allyl dextran and N,N'-methylenebisacrylamide can be preferably used as the gel matrix, and more preferably using Sephacryl (registered trademark) S-300, S-400, and S-500 chromatography to purify infectious HCV particles.

In ion-exchange chromatography, preferably, Q-Sepharose (registered trademark) can be used as an anion exchange resin. That is, HCV particles can be purified using Q-Sepharose, for example. Moreover, preferably, SP Sepharose (registered trademark) or the like is used as a cation exchange resin to purify HCV particles.

In affinity chromatography, a resin binding a substrate selected from among heparin, sulfated cellulofine, lectin, and various dyes as a ligand can be preferably used as a support to purify HCV particles. Further preferably, HCV particles can be purified with the use of a support binding HiTrap Heparin HP (registered trademark), HiTrap Blue HP (registered trademark), HiTrap Benzamidine FF (registered trademark), sulfated cellulofine, LCA, ConA, RCA-120, and WGA. The most preferable method is purification of HCV particles with the use of sulfated cellulofine as a support. HCV particles can be purified 30-fold or more in terms of the ratio of the HCV RNA copy number to the total protein amount in the solution before and after the purification.

In purification via density-gradient centrifugation, a sugar polymer, such as cesium chloride, sucrose, Nycodenz (registered trademark), Ficoll (registered trademark), or Percoll (registered trademark) can be preferably used as a solute that makes density gradient. Sucrose can be further preferably used. Preferably, water or a buffer, such as phosphate buffer, Tris buffer, acetate buffer, or glycine buffer, can be used as a solvent. The centrifugal force that is employed at the time of purification via density-gradient centrifugation is preferably ranges from 1 x 10⁴ g to 1 x 10⁹ g, more preferably ranges from 5 x 10⁴ g to 1 x 10⁷ g, and most preferably ranges from 5 x 10⁴ g to 5 x 10⁵ g.

Purification is carried out preferably at 0°C to 40°C, more preferably at 0°C to 25°C, and most preferably at 0°C to 10°C.

After concentration of HCV particles with an ultrafilter membrane, HCV particles can also be purified by density-gradient centrifugation. Furthermore, when purification is carried out via density-gradient centrifugation in combination with column chromatography, such techniques may be carried out in any combination in any order. Preferably, HCV particles are first purified through a plurality of chromatography columns followed by density-gradient centrifugation. More preferably, a fraction containing HCV particles obtained via anion exchange column chromatography followed by affinity chromatography is subjected to purification via density-gradient centrifugation. Most preferably, a fraction containing HCV particles obtained with the use of a Q-Sepharose (registered trademark) column is further purified with the use of a sulfated cellulofine-based column, and the resulting fraction containing HCV particles is then purified via density-gradient centrifugation. During the steps of column chromatography and density-gradient centrifugation, dialysis or ultrafiltration may be carried out to substitute a solute of a solution containing HCV particles and/or to concentrate HCV particles.

### (3) In activation of viral particles

The vaccine of the present invention reacts with the inactivated HCV particle as an antigen. With regard to the vaccine of the present invention, the presence or the absence of the infectivity of the HCV particles poses no problem for evaluation using rodents, since HCV does not exhibit infectivity to rodents. However, when the vaccine is inoculated to a human, the use of inactivated HCV particles is required. HCV particles can be inactivated by adding and mixing an inactivator such as formalin, β-propiolactone, or glutardialdehyde in, for example, a virus suspension to allow the inactivator to react with viruses (Appaiahgari et al., Vaccine, 22: 3669-3675, 2004). Further, HCV particles may be irradiated with ultraviolet rays to cause the loss of infectivity of viruses, and viruses can be immediately inactivated. Irradiation with ultraviolet rays is preferred since it realizes virus inactivation with little influence on proteins that constitute viruses. A source of ultraviolet rays used for inactivation can be a commercially available germicidal lamp. In particular, a 15w germicidal lamp can be used, although the source is not limited thereto. In the present invention, HCV particles that are purified by the method described above are used, and methods of inactivation are not limited by a purified or unpurified state. Preferably, a solution containing infectious HCV particles may be irradiated with ultraviolet rays at 20 mW/cm² at room temperature for at least 5 minutes to inactivate infectious HCV particles.

### (4) Adjuvant

Examples of an adjuvant that can be used herein include aluminium hydroxide (Alum) whose use as an adjuvant for vaccines has already been approved, and double-stranded RNA (dsRNA) and unmethylated CpG-containing oligonucleotide (CpG-ODN) for which clinical trials have been conducted.

Examples of dsRNA include polyI:C, polyICLC, and polyIpolyC12U.

Any immunostimulatory oligonucleotide to be contained in the vaccine of the present invention may be used herein as long as it contains unmethylated CpG. A preferable example thereof is an oligonucleotide disclosed in International Patent Publication WO07/139190. A further preferable example thereof is GGGGGGGCGACGATCGTCAGG (SEQ ID NO: 4, referred as Mod87).

Degradation of an oligonucleotide having the phosphodiester backbone is mediated by exonuclease and endonuclease. It is known that phosphorothioate modification of nucleotide-to-nucleotide bonds results in acquisition of resistance to these nucleases. Examples of an oligonucleotide in which some bonds between nucleotide residues are modified include oligonucleotides in which 5' and 3' terminal nucleotides are linked via phosphorothioate-modified bonds and oligonucleotides in which nucleotides of a polyG sequence are linked via phosphorothioate-modified bonds. These oligonucleotides have resistance to exonuclease.

Therefore, a more preferred example of an oligonucleotide is EEEEEEECGACGATCGTCAEG (SEQ ID NO: 5; phosphorothioated guanine (G) is denoted as "E" and referred to as "Mod87s"), wherein the phosphodiester bond between 5'-terminal polyG sequence and the end on the 3' terminal side of the oligonucleotide of SEQ ID NO: 4 has undergone phosphorothioate modification.

### (5) Vaccine composition and its effects

The hepatitis C virus vaccine composition according to the present invention can be produced by mixing inactivated hepatitis C virus (HCV) particles produced by the above-mentioned method or the like with adjuvants, particularly unmethylated CpG-containing oligonucleotide shown in SEQ ID NO: 5 in the sequence listing and aluminium hydroxide by a conventional method. The vaccine composition of the present invention contains inactivated HCV particles as antigens in an amount ranging from 0.001 to 99.999% by weight.

Also, dosage can be appropriately determined depending on subjects' age, patients' symptoms, therapeutic purposes, routes of administration, and the like. Any dosage may be employed herein, as long as the amount is sufficient for activation of immunity capable of preventing HCV infection or the onset of HCV. In general, dosage preferably ranges from 0.1 mg to 10000 mg, and more preferably ranges from 1 mg to 100 mg per administration to an adult. Also, after initial administration, specifically 2 weeks, 4 weeks, 6 weeks, 8 weeks, 2 months, 3 months, 4 months, 5 months, 6 months, or 1 year or more later, one or more booster doses of the vaccine composition of the present invention are provided. Thus, immune-response-inducing capacity can be maintained. Therefore, the frequency of administration is preferably 2 or more times, furthermore preferably ranges from 2 to 6 times, more preferably ranges from 3 to 5 times, and is most preferably 4 times.

The effects of the vaccine composition of the present invention can be evaluated by administering the vaccine to an animal and then detecting an anti-HCV antibody induced by the immune reaction. Animals to be used for immunization may be any animals, as long as they are non-human animals (e.g., non-human mammals) such as chimpanzees, monkeys, mice, rats, hamsters, and rabbits. In the present invention, such animals are preferably mice. Examples in which mice are used are as described below.

In general, a 4- to 10-week-old mouse is immunized by administering several times the HCV particles obtained by the above steps (1) to (3) as antigens and an adjuvant to the mouse. As such an adjuvant, Alum, CpG-ODN, and dsRNA described in (4) can be used independently or in combination.

After administration of the vaccine composition, blood is collected via tail vein of the mouse to which the vaccine has been administered. The antibody titers against the HCV proteins in serum are measured as described in (6) below, so that the degree of immune response induced by the vaccine composition can be measured. HCV is known to infect cells via the envelope proteins. Hence, it is preferred to measure the antibody titers against envelope proteins (e.g., E1 protein and E2 protein). Further preferably, the activity of inhibiting HCV infection is measured as described in (7) below, so that the effects of the vaccine composition can be examined.

### (6) Measurement of antibody titer

For evaluation of the effects of the vaccine composition according to the present invention, an HCV protein is fixed (immobilized) to a support. Next, serum is added, and then an antibody in serum and the immobilized antigen are caused to react for sufficient time under sufficient conditions for the formation of a complex. Subsequently, the thus generated complex is brought into contact with an antibody (secondary antibody) recognizing the antibody (in the serum) bound with an enzyme, a dye, or a radioisotope as a signal, so that a 2nd mixture is generated. The 2nd mixture is caused to react for sufficient time under sufficient conditions for the formation of an antibody-antigen complex. The presence of the antibody recognizing the HCV protein is detected by detecting the signal from the enzyme, dye, or radioisotope.

As such an HCV protein to be immobilized to a support, an HCV particle can be directly used. Alternatively, cDNA consisting of the nucleotide sequences of, in the HCV genome, a core protein coding region, an E1 protein coding region, an E2 protein coding region, a p7 protein coding region, an NS2 protein coding region, an NS3 protein coding region, an NS4A protein coding region, an NS4B protein coding region, an NS5A protein coding region, and/or an NS5B protein coding region can be used. Proteins encoded by these regions expressed in *Escherichia coli*, yeast, mammalian cells, insect cells, or the like can also be used. Furthermore, such proteins can be chemically synthesized and then used.

Methods for expressing the envelope proteins of the J6CF strain in mammalian cells are as described in JP Patent Application No. 2007-193413 and JP Patent Application No. 2008-254338. Specifically, when the initiator methionine of the amino acid sequence (NCBI Protein Accession No. AAF01178.1) of a full-length protein of the J6CF strain is regarded as No.1, the E1 protein of the J6CF strain starts from the 192nd amino acid residue and ends at the 383rd amino acid residue. The E2 protein of J6CF strain starts from the 384th amino acid residue and ends at the 750th amino acid residue of the above amino acid sequence. The portion ranging from the 353rd to the 383rd amino acid residues of the E1 protein and the portion ranging from the 722nd to the 750th amino acid residues of the E2 protein are thought to be transmembrane domains (Cocquerel, L. et al., J. Virol. 74: 3623-3633, 2000).

For secretory expression of proteins in a culture supernatant by mammalian cells, the proteins are required to have a signal peptide but no transmembrane domain.

Therefore, in the case of the E1 protein of the J6CF strain, a sequence ranging from the 192nd to the 352nd amino acid residues can be used as such a protein having no transmembrane domain. In the case of the E2 protein of the same, a sequence ranging from the 384^{th} to the 720th amino acid residues can be used as such a protein having no transmembrane domain.

For secretory expression of the E1 or E2 protein having no transmembrane domain by mammalian cells, a nucleic acid encoding the protein is ligated downstream of a nucleic acid encoding a signal peptide, such that the reading frames (readable frames) for codons match. Furthermore, a stop codon is added to the 3' end, and then the resultant is inserted to an expression vector. A signal peptide consists mainly of hydrophobic amino acids; that is, 15 to 30 amino acid residues existing on the N terminus of a secretory protein, and is involved in the mechanisms of protein transport through the cell membrane

A signal peptide that can be used for secretory expression of a protein by mammalian cells may be of any secretory protein. Examples of a vector having a signal peptide include a vector having the signal peptide sequence of mouse GM-CSF (JP Patent Publication (Kokai) No. 63-276490 A (1988)), a pSecTag/FRT/V5-His vector (Invitrogen) having the signal peptide sequence of IgG κ chain, a p3XFLAG-CMV13 vector (Sigma) having the signal peptide sequence of preprotrypsin, a pFUSE-Fc2 vector (InvivoGen) having the signal peptide sequence of IL-2, and a pTriEx-7 vector (Novagen) having the signal peptide sequence of IgM.

When proteins are expressed, such proteins are expressed as fusion proteins of target proteins and label proteins, and fusion proteins can be detected or purified with the use of an antibody reacting with the label protein or a molecule that specifically binds thereto. Such label proteins are also referred to as "tags." Label proteins are not limited, and examples thereof include FLAG peptide (also referred to as flag peptide or Flag peptide), 3 x FLAG peptide (also referred to as 3 x FLAG peptide, 3 x Flag peptide, or 3 x flag peptide), HA peptide, 3 x HA peptide, myc peptide, 6 x His peptide, GST polypeptide, MBP polypeptide, PDZ domain polypeptide, alkaline phosphatase, immunoglobulin, and avidin. Such peptides or polypeptides are generally fused to the N- or C-terminus of the target proteins, but such peptides or polypeptides can be inserted into the target proteins according to need. A vector having a fusion polypeptide of a preprotrypsin signal peptide and a 3 x FLAG peptide is available as the p3xFLAG-CMV-9 vector from Sigma.

### (7) Measurement of inhibition of infection

To measure whether or not an animal serum sample obtained after administration of the vaccine composition has the activity of inhibiting HCV infection, infectious HCV-like particles (HCVpp) or infectious HCV particles (HCVcc) can be used.

Infectious HCV-like particles can be prepared by causing retrovirus particles to present functional HCV envelope proteins thereon. A green fluorescent protein marker gene or a luciferase gene is packaged within these infectious HCV-like particles, making it possible to rapidly measure with high reliability infection mediated by HCV envelope proteins (Bartosch, B. et al. J. Exp. Med. 197: 633-642, 2003).

Specifically, for example, a pcDNA J6dC-E2 vector is constructed by cloning a nucleic acid that encodes the 132nd to the 750th amino acid residues (a part of the core protein, the E1 protein, and the E2 protein) of the protein of J6CF (NCBI Protein Accession No. AAF01178.1) which is an HCV strain of genotype 2a into pcDNA3.1. A Gag-Pol 5349 expression vector is constructed by cloning genes that encodes gag and pol of a mouse leukemia virus into the vector. A Luc126 retrovirus vector is constructed by cloning a luciferase gene into the vector. The vectors are transfected into 293T human embryo renal cells (ATCC CRL-1573) using FuGENE6, so that HCVpp can be produced. After transfection, a culture solution containing pseudo particles is collected and then filtered with a 0.45-µm membrane filter, so that it can be used as an HCVpp sample.

For preparation of HCVpp having envelope proteins of genotype 1a, a pcDNA H77dC-E2 vector constructed by cloning a nucleic acid that encodes the 132nd to the 746th amino acid residues (a part of the core protein, the E1 protein, and the E2 protein) of the protein (NCBI Protein accession No. AAB67036.1) of H77 (HCV of genotype 1a) into pcDNA3.1 can be used. For preparation of HCVpp having envelope proteins of genotype 1b, a pcDNA THdC-E2 vector constructed by cloning a nucleic acid encoding the 132nd to the 747th amino acid residues (a part of the core protein, the E1 protein, and the E2 protein) of a protein of TH (HCV of genotype 1b) (Wakita, T. et al., J. Biol. Chem., 269, 14205-14210, 1994) into pcDNA3.1 can be used.

For example, HCVpp is mixed with a diluted serum sample, and then the mixture is allowed to react at room temperature for 30 minutes. Serum is diluted with a DMEM medium (DMEM containing 10% FCS (Invitrogen), a 1% MEM nonessential amino acid solution (Invitrogen), 10 mM HEPES-Tris (pH 7.3), and 1 mM sodium pyruvate). The above mixture of HCVpp and the serum sample is added to Huh7.5.1 cells cultured on the previous day in 96-well plate at 1 x 10⁴ cells/well, followed by 3 hours of culture at 37°C. After culture, the sample is removed, cells are washed once with PBS, a fresh medium is added, and then cells are cultured continuously. After 72 hours, a culture supernatant is removed, washing is performed 4 times with PBS, 25 µL/well DMEM medium and 25 µL/well Steady-Glo (Promega: catalog No. E2520) are added, and then cells are lysed according to instructions included therewith. The cell lysis solution (40 µL/well) is transferred to a white 96-well plate (Sumitomo Bakelite Co., Ltd.: catalog No. MS-8496W), and then luminescence intensity is measured using ARVO X4 (PerkinElmer).

A value obtained after mixing with DMEM is regarded as 100% infection. Luciferase activity after mixing with a serum sample is expressed with percentages (%) and thus the activity of inhibiting HCV infection (%) can be found.

Also, for example, HCVcc is mixed with a diluted serum sample, and then the mixture is allowed to react at room temperature for 30 minutes. Serum is diluted with a DMEM medium (DMEM containing 10% FCS (Invitrogen), a 1% MEM nonessential amino acid solution (Invitrogen), 10 mM HEPES-Tris (pH 7.3), and 1 mM sodium pyruvate). The mixture of HCVcc such as J6/JFH1-HCV particles and the serum sample is added to Huh7.5.1 cells cultured on the previous day in a 96-well plate at 1 x 10⁴ cells/well, followed by 3 hours of culture at 37°C. After culture, the sample is removed, cells are washed once with PBS, a fresh medium is added, and then cells are cultured continuously. After 72 hours, a culture supernatant is removed and then the plate is soaked in ice-cooled methanol, so as to immobilize cells. Subsequently, methanol is removed by air drying and then cells are solubilized using BlockAce (registered trademark) (Dainippon Pharmaceutical) containing 0.3% Triton (registered trademark)-X 100 (GE Healthcare). An HRP-labeled anti-HCV-core antibody (Ortho Clinical Diagnostics) is reacted, and then hepatitis C virus-infected cells are detected using a QuantaBlu (registered trademark) (PIERCE) reaction solution. After addition of a QuantaBlu stop solution, 20 µL/well of the resultant is transferred to a black 384-well plate (Corning: catalog No. 3676), and then fluorescence intensity is measured using ARVO X4 (PerkinElmer).

The value obtained after mixing with DMEM is regarded as representing 100% infection. QuantaBlu fluorescence intensity after mixing with a serum sample is expressed with percentages (%), and thus the activity of inhibiting HCV infection (%) can be found.

As confirmed above, the vaccine composition according to the present invention has significantly strong activity of inhibiting HCV infection. Moreover, the vaccine composition is advantageous in that it can exhibit strong activity of inhibiting HCV infection not only for HCV of genotype 2a used as an antigen, but also for HCVs of other types of genotype (e.g., 1a, 1b, and 2b).

### Examples

The present invention will be described in more detail with reference to the examples set forth below.

### Example 1

### Preparation of J6/JFH1-HCV particles

cDNA (genomic cDNA) was obtained by reverse transcription of the full genomic RNA region of the hepatitis C virus (HCV) JFH1 strain (genotype 2a) separated from a fulminant hepatitis patient. The cDNA was cloned downstream of the T7 RNA promoter sequence of a pUC19 plasmid, so as to obtain a pJFH1 plasmid DNA (Wakita, T. et al., Nat. Med., 11 (2005) p.791-796 and International Patent Publication WO 2004/104198). The pJFH1 plasmid DNA was digested with *Eco*R I and then partially digested with *Bcl* I. A fragment (about 2840 bp) ranging from the *EcoR* I site to the first *Bcl* I site was excised so as to prepare a plasmid DNA fragment, and then the plasmid DNA fragment was purified.

Meanwhile, HCV J6CF-strain-derived genomic cDNA (GenBank Accession No. AF177036, Yanagi, M., et al., Virology 262: 250-263 (1999)) was cloned downstream of the T7 RNA promoter sequence of a pUC19 plasmid, so as to obtain a pJ6CF plasmid DNA (International Patent Publication WO 2006/022422). The pJ6CF plasmid DNA was partially digested with *Eco*R I and *Bcl* I, and then the thus obtained about 2840-bp fragment was purified. The fragment was ligated to the pJFH1 plasmid fragment obtained by excision of the *EcoR* I-*Bcl* I fragment, so that a pJ6/JFH1 plasmid DNA was prepared. The DNA (SEQ ID NO: 2) cloned into pJ6/JFH1 is the genomic cDNA of a chimera virus prepared by ligating a sequence encoding the region ranging from the 17th amino acid residue to the C terminal amino acid residue of the NS2 protein, sequences encoding NS3, NS4A, NS4B, NS5A, and NS5B proteins in this order, and the 3' untranslated region of the genomic cDNA of the JFH1 strain to the 5' untranslated region, sequences encoding core, E1, E2, and p7 proteins, and the sequence encoding the region ranging from the N terminal amino acid residue to the 16th amino acid residue of the NS2 protein in the genomic cDNA of the J6CF strain.

After cleavage of the pJ6/JFH1 with *Xba* I, Mung Bean Nuclease 20U (total reaction solution volume: 50 µL) was added followed by 30 minutes of incubation at 30°C, so as to blunt-end the terminus cleaved with *Xba* I. Subsequently, phenol chloroform extraction and ethanol precipitation were performed, so that a fragment cleaved with *Xba* I, from which 4 bases (CTAG) had been removed, was obtained. RNA was synthesized using the DNA as a template and a MEGA script T7 kit (Ambion). The thus synthesized HCV RNA was used for the following introduction into cells.

3 x 10⁶ Huh-7 cells and 5 µg of HCV RNA were suspended in a Cytomix solution (120 mM KCl, 0.15 mM CaCl₂, 10 mM K₂HPO₄/KH₂PO₄, 25 mM Hepes, 2 mM EGTA, 5 mM MgCl₂, 20 mM ATP, 50 mM glutathione 400 µL). After the suspension was transferred to a 4-mm cuvette, HCV RNA was electroporated into Huh-7 cells using a Gene Pulser (BioRad) at 260 V and 950 µF. Subsequently, cells into which the HCV RNA had been introduced were seeded on a 10-cm² dish, and then subcultured. Upon subculture, the HCV core protein contained in culture supernatants was quantified using the HCV antigen ELISA test kit (Ortho Clinical Diagnostics) to confirm the production of HCV particles. Culture supernatants containing the core protein at high levels and exhibiting high activity of producing HCV particles were selected and then stored as virus stocks.

About 100 µL each of the above obtained J6/JFH1 virus stock (4 x 10⁴ ffu/mL) was added to Huh-7 cells cultured with 10% FCS-DMEM medium (1% MEM nonessential amino acid solution (Invitrogen), 10 mM HEPES-Tris (pH 7.3), 1 mM sodium pyruvate) in the 10-cm dish, so that Huh-7 cells were infected with the HCV virus.

The cells were adequately subcultured while avoiding cells from becoming confluent, and then culture expansion was performed from one to six 225-cm² flasks. Subsequently, cells were detached from five 225-cm² flasks, seeded in three 5-layer Cellstacks (registered trademark) (Corning), a medium was added thereto in an amount of 650 mL/cell stack. The cells obtained from the other 1 flask were seeded in 6 flasks and thus virus production could be efficiently continued.

On the day following subculture, the media were discarded and 650 mL of 2% FCS-DMEM medium (1% MEM nonessential amino acid solution (Invitrogen), 10 mM HEPES (pH 7.3), 1 mM sodium pyruvate) was added. The media were recovered 3 days after medium exchange, and then caused to pass through a 0.45-µm filter. The resultants were stored in a deep freezer. Also, 650 mL of 2% FCS-DMEM medium (1% MEM non-essential amino acid solution, 10 mM HEPES (pH 7.3), and 1 mM sodium pyruvate) was added to the Cellstacks after the culture supernatants had been recovered, and culture was continued. A similar procedure was repeated 2 days after the medium exchange and the culture supernatants were recovered. A similar procedure was then repeated one more time. The culture supernatants recovered herein were used as culture supernatants containing J6/JFH1-HCV particles in the Examples below.

### Example 2

### Purification of J6/JFH1-HCV particles

J6/JFH1-HCV particles produced in Example 1 were purified via the following 3 steps.

### 1) Concentration and diafiltration

The culture supernatants containing HCV particles obtained in Example 1 were concentrated 60-fold using a Hollow Fiber Cartridge (GE Healthcare: 500 kDa cut-off model No. UFP-500-C-8A, hereinafter, referred to as "Hollow Fiber").

### 2) Density-gradient ultracentrifugation

To the Ultra-clear 25 mm x 89 mm centrifuge tube (Catalog No. 344058, Beckman Coulter), 3 mL of TNE buffer containing cold 60% sucrose (10 mM Tris-HCl (pH 7.4), 150 mM NaCl, 1 mM EDTA) was added, and 7 mL of TNE buffer containing 20% sucrose was overlaid thereon. Further, 25 mL of the sample was overlaid onto the TNE buffer containing 20% sucrose. Ultracentrifugation was carried out using a SW-28 (Beckman Coulter) at 28,000 rpm for 4 hours at 4°C.

The bottom of the tube was perforated using the 25G injection needle (Terumo) and twelve 1-mL fractions were obtained. Specific gravity was measured for the solution of each fraction, so that the formation of the density gradient of sucrose was confirmed. Fractions with 3rd, 4th, and 5th largest specific gravity were recovered in descending order and then used for concentration and buffer exchange.

### 3) Concentration and buffer exchange

The elution fraction was subjected to buffer exchange and concentration using Amicon Ultra-15 Centrifugal Filter Units (molecular weight to be excluded: 100 kDa, Millipore) and TNE buffer. The thus-obtained concentrate was used as a virus solution containing infectious HCV particles in the immunization step described below.

### Example 3

### Inactivation of HCV particles

The concentrated hepatitis C viruses obtained by steps 1) to 3) in Example 2 were inactivated via ultraviolet irradiation. As a source of ultraviolet rays, a GL-15 (Toshiba) was used. Specifically, the solution containing purified hepatitis C virus particles (the JFH-1 strain) having an infectious titer of 1 x 10⁶ ffu/mL was introduced into a silicon-coated polyethylene Eppendorf tube (Assist Co., Ltd), the tube was placed at a distance from the source of ultraviolet rays, so that the ultraviolet rays would be applied at the intensity of 20 mW/cm², and UV-C was applied for 5 minutes.

After ultraviolet irradiation, hepatitis C virus particles were serially diluted 50-fold, 250-fold, 1,250-fold, 6,250-fold, 31,250-fold, 156,250-fold, and 781,250-fold in Dulbecco's modified Eagle medium (DMEM).

On the previous day, the Huh-7 cells were seeded on a 96-well poly-L-lysine-coated plate (Corning 96 Well Clear Flat Bottom Poly-L-Lysine Coated Microplate) at 1 x 10⁴ cells/well, the serially-diluted viral particles were seeded thereonto, and culture was conducted at 37°C for 72 hours.

After the culture supernatant was removed, the plate was soaked in ice-cold methanol to fix the cells. Thereafter, methanol was removed via air drying, and cells were solubilized with the use of Block Ace (registered trademark) (Dainippon Pharmaceutical Co., Ltd.) containing 0.3% Triton (registered trademark)-X 100 (GE Healthcare). HCV-infected cells were detected using the clone 2H9 anti-HCV-core antibody (Wakita, T. et al., Nat. Med. 11: 791-796, 2005) and goat anti-mouse IgG-Alexa488 (Molecular Probes), and the HCV-infected cells were counted under a fluorescent microscope (IX-70; Olympus). The infectious titer of the ultraviolet-irradiated hepatitis C viruses was found to be the same or below the detection limit. HCV particles that had been caused to completely lose infectivity were administered to mice in the Examples below.

### Example 4

### Preparation of J6E1Fc protein and J6E2Fc protein

The J6E1Fc protein and the J6E2Fc protein were prepared by the following method.

### (1) Construction of vector for expression of fusion protein of E1 protein derived from J6CF strain of HCV2a added to Fc protein of human IgG

A gene encoding the E1 protein consisting of a region ranging from the 192nd to the 353rd amino acid residues of the precursor protein of the J6CF strain, when the initiation methionine at the N-terminus was designated as the 1st amino acid residue, was amplified by a PCR method using the cDNA of the genomic RNA of the J6CF strain of HCV2a (GenBank Accession No. AF177036) as a template, an Advantage GC1 PCR kit (Takara Bio Inc.), and J6E1F-s (SEQ ID NO: 9: CACAAGCTTGCCGAAGTGAAGAACATCAGT) and J6E1F-as (SEQ ID NO: 10: TCGGGATCCCAATGAGCCCCGCTAATGATGTC) as primers.

Next, the fragment amplified by the PCR method was cloned into pCR-TOPO (Invitrogen) and then 3 clones were subjected to sequence analysis. Clones with correct nucleotide sequences were designated as "pTOPO-J6E1F."

Next, p3XFLAG-CMV-13 (Sigma) was digested with *Hind* III and *BamH* I, separated by agarose gel electrophoresis, and then purified. The thus obtained DNA fragment and a DNA fragment containing cDNA encoding the E1 protein of J6CF (obtained by digestion of pTOPO-J6E1F with *Hind* III and *BamH* I) were cyclized with T4 DNA ligase. The vector was designated as "CMV-13-J6E1F." CMV-13-J6E1F was digested with *Sac* I and *BamH* I, the DNA fragment encoding the E1 protein of JFH1 was separated by agarose gel electrophoresis and then purified using GeneElute (Sigma).

A CDM-mIL7R-Ig vector (Sudo et al., Proc Natl. Acad Sci U.S.A., 1993, Vol. 90, p. 9125-9129) expressing a chimeric protein comprising a mouse IL-7 receptor-human immunoglobulin Fc domain was digested with *Sac* I and *Bam*H I. A DNA fragment containing the sequence encoding a human immunoglobulin Fc region was separated by agarose electrophoresis and then purified. The DNA fragment and the DNA fragment encoding the above J6E1 protein were linked with T4 DNA ligase. Thus, a "CDM-J6E1Fc" vector expressing the chimeric protein (hereinafter, referred to as "J6E1Fc protein") consisting of the J6E1 protein and the immunoglobulin Fc domain was obtained.

### (2) Construction of vector for expression of fusion protein of E2 protein derived from the J6CF strain of HCV2a added to Fc protein of human IgG

First, a gene encoding a protein consisting of a region corresponding to the 384th to 720th amino acid residues of the precursor protein of the J6CF strain, when the initiation methionine at the N-terminus was designated as the 1st amino acid, was amplified by a PCR method using the cDNA (GenBank Accession No. AF177036) of the genomic RNA of the J6CF strain of HCV2a as a template, an Advantage GC2 PCR kit (Takara Bio Inc.), and J6E2Fc-s (SEQ ID NO: 6: CACAAGCTTCGCACCCATACTGTTGGGG) and J6E2Fc-as (SEQ ID NO: 7: ACAGGATCCCATCGGACGATGTATTTTGTG) as primers.

Next, the thus amplified DNA fragment was cloned into pCR-TOPO (Invitrogen) and then 3 clones were subjected to sequence analysis. A gene fragment encoding the antigen E2 protein was digested with *Hind* III and *BamH* I and thus excised from clones having the correct nucleotide sequence insert and then inserted in-frame between the *Hind* III site and the *BamH* I site downstream of a signal peptide sequence of p3xFLAG-CMV-13 (Sigma). The vector was designated as CMV-13-J6E2.

Subsequently, CMV-13-J6E2 was digested with *Sac* I and *BamH* I. The thus generated DNA fragments encoding the signal peptide sequence and the antigen E2 protein, respectively, were each separated by agarose gel electrophoresis, and then purified using GeneElute (Sigma).

Thereafter, DNA fragments encoding the above signal peptide sequence and the antigen E2 protein, respectively, were inserted in-frame between the *Sac* I site and the *BamH* I site of the CDM-mIL7R-Ig vector (Sudo et al., Proc Natl. Acad Sci U.S.A., 1993, Vol. 90, p. 9125-9129) expressing the chimeric protein comprising mouse IL-7 receptor-human immunoglobulin Fc domain. Thus a CDM-J6E2Fc vector expressing the antigen E2 protein to which the human immunoglobulin Fc domain had been added (hereinafter, J6E2Fc protein) was obtained.

### (3) Expression of fusion protein of HCVE1 protein or HCVE2 protein with IgGFc protein

CDM-J6E1Fc or CDM-J6E2Fc was introduced into COS1 cells derived from monkey kidney and then each fusion protein was expressed as described below.

First, COS1 cells were subcultured in RPMI1640 medium (Invitrogen) containing 10% fetal calf serum (Invitrogen), 100 U/mL penicillin, and 100 µg/mL streptomycin. On the day before the gene transfer, COS1 cells were seeded in 150 cm² culture flasks (Corning Coaster Corporation) at a dilution ratio of 1 : 2 and then cultured overnight at 37°C in a 5% CO₂ incubator.

Subsequently, DEAE dextran (GE Healthcare) and chloroquine (Sigma) were added to RPMI1640 medium at final concentrations of 400 µg/mL and 100 µM, respectively. 50 µg of the above expression vector (CDM-J6E1Fc or CDM-J6E2Fc) was added at a concentration of 0.1 µg/µL to 13 mL of the solution and then cells were cultured for 3 to 4 days.

After culture, the supernatant of COS1 cells was aspirated off. 10 mL of PBS(-) (Nissui Pharmaceutical Co., Ltd.) was added, and again, PBS(-) was aspirated off for washing cells. Subsequently, a DEAE dextran-DNA mixture was added at 13 mL/150 cm² flask and then the resultant was left to stand at 37°C in the presence of 5% CO₂.

Four hours later, the DEAE dextran-DNA mixture was aspirated off, each flask was washed once with 10 mL of PBS, Hybridoma-SFM medium (Invitrogen) was added at 50 mL/flask, and then cells were cultured at 37°C in the presence of 5% CO₂ for 4 days. Thereafter, the culture supernatant was collected in a 50-mL centrifuge tube (Corning Coaster Corporation) and then centrifuged at 2500 rpm for 30 minutes at 4°C. The supernatant was filtered through a 0.2-µm filter (Whatman).

### (4) Purification of fusion protein of HCVE1 protein or HCVE2 protein with IgGFc protein

The culture supernatant of COS1 cells into which CDM-J6E1Fc or CDM-J6E2Fc had been introduced was subjected to purification using Prosep-A (Millipore) as a carrier to which Protein-A had been bound, as described below.

First, an Econocolumn was filled with 1 mL of Prosep-A, 500 mL of the culture supernatant was caused to pass through at a flow rate of 1-1.5 mL/min, and then washed with 20 mL of PBS(-).

Next, 5 fractions were eluted with 0.1 M Glycine-HCl (pH 3.0) to 1 mL/fraction. Immediately after elution, 1 M Tris-HCl (pH 9.5) was added to return the pH to neutral. 20 µL of each fraction was fractionated under reductive conditions by SDS-polyacrylamide gel electrophoresis, and then stained with Coomassie brilliant blue. As a result, the fusion protein (J6E1Fc protein) consisting of the HCVE1 protein and the human immunoglobulin Fc domain or the fusion protein (J6E2Fc protein) consisting of the HCVE2 protein and the human immunoglobulin Fc domain was purified. The molecular weights under reductive conditions were revealed to be about 60 kDa and about 97 kDa, respectively.

### Example 5

### Preparation of vaccine composition containing antigen and adjuvant

A vaccine composition was prepared as an emulsion as described below.

Alum, unmethylated CpG-ODN, and polyI:C were used as adjuvants. As Alum, Imject Alum (registered trademark) (PIERCE: catalog No. 77161) was used. As unmethylated CpG-ODN, Mod87s (SEQ ID NO: 5) was used. polyI:C was purchased from Yamasa Shoyu. These adjuvants were used independently or in combination.

When the adjuvants were used independently, Alum was prepared at 200 µg/100 µL, Mod87s was prepared at 25 µg/100 µL, and polyI:C was prepared at 100 µg/100 µL. Also, when the adjuvants were used in combination (Alum+Mod87s, Alum+poly I:C, or Mod87s+poly I:C), Alum was prepared at 200 µg/50 µL, Mod87s was prepared at 25 µg/50 µL, and polyI:C was prepared at 100 µg/50 µL, and thus two of the three adjuvants were combined and used.

When the J6E2Fc protein described in Example 4 was used as an antigen, 50 µL of Alum and Mod87s were added to 100 µL of 0.2 µg/µL J6E2Fc protein solution (containing 20 µg of the J6E2Fc protein), so that an emulsion was formed. In addition, when 5 µg of the J6E2Fc protein was used as an antigen, 75 µL of PBS was added to 25 µL of a 0.2 µg/µL J6E2Fc protein solution to 100 µL and then similarly mixed with an adjuvant(s).

When an adjuvant was used independently for 100 µL of a solution containing inactivated J6/JFH1-HCV particles (in an amount equivalent to 2 pmol of HCV core) described in Example 3 to be used as an antigen, Alum, Mod87s, or poly I:C in an amount equivalent thereto was added, so as to form an emulsion. Also, when the adjuvants were used in combination, two adjuvants from among 50 µL of Alum, 50 µL of Mod87s, and 50 µL of poly I:C were combined (a total of 100 µL) and then the combination was added to 100 µL of a solution containing inactivated J6/JFH1-HCV particles, so as to form an emulsion.

### Example 6

### Evaluation of the immune-response-inducing capacity of vaccine composition

### 1. Administration of vaccine composition

### 1) Administration of J6E2Fc chimeric protein

Balb/c mice (5-week-old, female) were immunized via intraperitoneal administration of an emulsion containing the J6E2Fc protein (5 µg or 20 µg) prepared in Example 5. Two weeks later, a similarly prepared emulsion containing the J6E2Fc protein was similarly administered intraperitoneally for further immunization. Serum samples were prepared from blood collected on day 10 after the final immunization.

In addition, saline was administered as a control.

### 2) Administration of inactivated J6/JFH1-HCV particles

Balb/c mice (5-week-old, female) were immunized via intraperitoneal administration of an emulsion containing inactivated J6/JFH1-HCV particles (in an amount equivalent to 2 pmol of HCV core) prepared in Example 5. Two weeks later, a similarly prepared emulsion containing inactivated J6/JFH1-HCV particles was similarly administered intraperitoneally for further immunization. Furthermore, 4 weeks later and 6 weeks later, intraperitoneal administration was similarly performed for immunization. Serum samples were prepared from blood collected 1 week after the final immunization (7 weeks after the primary immunization).

In addition, saline was administered as a control.

### 2. Measurement of antibody titers against E1 protein and E2 protein

Antibody titers against the E1 protein and the E2 protein in the serum samples of mice to which each vaccine composition had been administered as described in "1. Administration of vaccine composition" above were measured. Antibody titers were measured by immobilizing the E1 protein or the E2 protein on a plate and determining by EIA whether antibodies in the serum of each mouse inoculated with each vaccine bound to the E1 protein or the E2 protein immobilized on the plate.

### 1) Preparation of J6CF strain-E2 protein

The E2 protein of the J6CF strain was prepared as follows. The gene encoding the E2 protein having no transmembrane region and corresponding to the 384th to the 720th amino acid residues when the N-terminal initiation methionine of the J6CF full-length protein sequence (the protein sequence encoded by the genome sequence of the J6CF strain; the amino acid sequence under GenBank Accession No. AF177036) was regarded as the 1st amino acid residue was amplified by a PCR method using genomic cDNA derived from the J6CF strain of genotype 2a (GenBank Accession No. AF177036) as a template, an Advantage GC2 PCR kit (Takara Bio Inc.), and J6E2Fc-s (SEQ ID NO: 6: CACAAGCTTCGCACCCATACTGTTGGGG) and J6E2dTM-as (SEQ ID NO: 8: GCTCTAGATTACCATCGGACGATGTATTTTGT). The amplified DNA fragment was cloned into pCR-TOPO (Invitrogen) and then 3 clones were subjected to nucleotide sequence analysis. Clones with correct nucleotide sequence inserts were designated as "pTOPO-J6E2dTM."

Subsequently, DNA obtained by digesting p3xFLAG-CMV-9 (SIGMA) with *Hin*d III and *Xba* I was ligated to the DNA fragment of approximately 1,000 bp excised from pTOPO-J6E2dTM with *Hind* III and *Xba* I for cyclization with the aid of T4 DNA ligase. The resulting vector was designated as CMV-3XFLAGJ6E2dTM.

CMV-3XFLAGJ6E2dTM was introduced into COS1 cells derived from the monkey kidney (Accession Number RCB0143, obtained from Riken Cell Bank) to express proteins therein as described below.

The COS1 cells were cultured in Dulbecco's MEM (D-MEM: Invitrogen) containing 10% fetal calf serum (Invitrogen), 100 U/mL penicillin, and 100 µg/mL streptomycin sulfate. The COS1 cells were seeded in a 150-cm² culture flask (Corning Coaster) at a dilution ratio of 1:2 on the previous day of gene transfer, and the cells were cultured at 37°C in a 5% CO₂ incubator overnight.

Separately, DEAE dextran (Pharmacia) and chloroquine (SIGMA) were added to D-MEM medium (Invitrogen) to result in final concentrations of 400 µg/mL and 100 µM, respectively. Furthermore, 50 µg of the CMV-3XFLAGJ6E2dTM expression vector was added at a concentration of 0.1 µg/µL to 13 mL of the solution, and culture was then performed. Subsequently, the supernatant of the cultured COS1 cells was aspirated off, and 10 mL of PBS (-) (Nissui) was added thereto and the cells were washed once. After PBS(-) was aspirated off, 13 mL of the DEAE dextran-DNA mixture was added to each 150-cm² flask, and incubation was then carried out at 37°C in the presence of 5% CO₂ for 4 hours.

Four hours later, the DEAE dextran-DNA mixture was aspirated off, and the cells were washed once with 10 mL of PBS. CHO-SFM medium (Invitrogen) was added thereto in amounts of 50 mL per flask, and culture was performed at 37°C in the presence of 5% CO₂. The culture supernatant was collected in a 50-mL centrifuge tube (Corning Coaster) 4 days later. The collected supernatant was centrifuged at 6,000 rpm (with the use of a HITACHI RPR9-2 rotor) for 30 minutes at 4°C and filtered through a 0.2-µm filter (Whatman).

The filtered culture supernatant was purified with the use of anti-FLAG M2 agarose (SIGMA) as follows. To 500 mL of the culture supernatant, 1 mL of anti-FLAG M2 agarose was added, and the reaction was allowed to proceed at 4°C (in a low-temperature chamber) for 2 hours while undergoing agitation in a spinner bottle. A mixture of the supernatant and anti-FLAG M2 agarose was transferred to the Econo-Column (BIO-RAD) 2 hours later, and flow-through fractions were collected. Subsequently, the column was washed twice with 10 mL of TBS (50 mM Tris-HCl, 150 mM NaCl, pH 7.4). Six fractions (1 mL of each fraction) were eluted with the use of 0.1M glycine-HCl (pH 3.5). Immediately after elution, 1M Tris-HCl (pH 9.5) was added for neutralization. The fractions (20 µl each) were subjected to SDS-polyacrylamide gel electrophoresis under reductive conditions and stained with Coomassie brilliant blue. As a result, E2 proteins derived from the J6CF strain were confirmed to be purified. The protein is designated as "J6E2dTM protein."

### 2) Measurement of antibody titer against envelope protein in serum sample

Serum obtained via blood collection from mice to which each vaccine composition was administered in "1. Administration of vaccine composition" above were subjected to measurement of antibody titers against the E1 protein and the E2 protein of the J6CF strain by the following method. The J6E1Fc protein was used as an antigen for antibody titer against the J6CF-strain-derived E1 protein. The J6E2Fc protein or the J6E2dTM protein was used as an antigen for antibody titer against the J6CF-strain-derived E2 protein. After dilution of such an antigen with PBS to 1 µg/mL, the solution was added at 50 µL/well to an immunoplate (Nunc: catalog No. 439454), and then incubated at room temperature for 2 hours or overnight at 4°C for immobilization. The antigen solution was discarded, Blocking One (NACALAI TESQUE, INC.: catalog No. 03953-95) diluted 5-fold with MilliQ water was added at 200 µL/well, and then incubation was performed at room temperature for 1 hour or overnight at 4°C, so that blocking was performed. After the completion of blocking, washing was performed twice with 0.05% Tween20/PBS (150 µL/well), and then serum diluted 1000- to 10000-fold with 0.05% Tween20/PBS was added at 50 µL/well, followed by 1.5 hours of reaction at room temperature. After completion of the reaction, the resultant was washed 3 times with 0.05% Tween20/PBS 150 µL/well, and then horseradish peroxidase conjugated anti-mouse IgG (GE Healthcare) diluted 5000 fold with 0.05% Tween20/PBS was added at 50 µL/well, followed by 1 hour of reaction at room temperature. After completion of the reaction, the resultant was washed 4 times with 0.05% Tween20/PBS, a staining solution (containing a substrate solution in an amount 1/100 of the solution) of a peroxidase staining kit (Sumitomo Bakelite Co., Ltd.: catalog No. ML-1120T) was added at 50 µL/well, and then incubation was performed at room temperature for 5 minutes to 15 minutes. A stop solution was added at 50 µL/well to stop the reaction, and then absorbance at 450 nm was measured using Benchmark Plus (Bio-Rad).

### 3. Measurement of neutralization titer

Effects of vaccines were examined by determining whether or not antibodies in serum samples obtained via blood collection from mice to which each vaccine composition of "1" above had been administered were able to inhibit infection with HCVpp or HCVcc.

### 1) Preparation of infectious HCV-like particles (HCVpp)

HCVpp was prepared according to the method of Bartosch et al (document: Bartosch, B. et al. (2003) J. Exp. Med., 197, 633-642). This is a method for preparing a pseudo virus expressing HCV envelope proteins on its surface by which 3 types of vector (a vector expressing retrovirus Gag-pol, a vector expressing HCV envelope proteins, and a retrovirus packaging vector expressing a reporter gene) are expressed in animal cells, so that the reporter gene is packaged.

For preparation of HCVpp having envelope proteins of genotype 2a, a nucleic acid encoding the 132^{nd} to 750^{th} amino acid residues (a part of the core protein, the E1 protein, and the E2 protein) of the protein of the J6CF strain of HCV of genotype 2a (NCBI Protein Accession No. AAF01178.1) was cloned into pcDNA3.1 and then the resulting pcDNA J6dC-E2 expression vector was used. For preparation of HCVpp having envelope proteins of genotype 1a, a nucleic acid encoding the 132^{nd} to the 746^{th} amino acid residues (a part of the core protein, the E1 protein, and the E2 protein) of the protein of H77 of HCV of genotype 1a (NCBI Protein Accession No. AAB67036.1) was cloned into pcDNA3.1, and then the resulting pcDNA H77dC-E2 expression vector was used. For preparation of HCVpp having envelope proteins of genotype 1b, a nucleic acid encoding the 132^{nd} to the 747^{th} amino acid residues (a part of the core protein, the E1 protein, and the E2 protein) of the protein of TH of HCV of genotype 1b (Wakita, T. et al., J. Biol. Chem., 269: 14205-14210, 1994, Moradpour, D. et al., Biochem. Biophys. Res. Commun., 246: 920-924, 1998, and International Patent Publication WO2006/022422) was cloned into pcDNA3.1, and then the resulting pcDNA THdC-E2 expression vector was used.

Gag-Pol 5349 was used as an expression vector constructed by cloning genes encoding gag and pol of a mouse leukemia virus. Luc 126 was used as a retrovirus packaging vector constructed by cloning a luciferase gene.

293T cells were subcultured in 10% FCS-DMEM medium (1% MEM nonessential amino acid solution (Invitrogen), 10 mM HEPES (pH 7.3), 1 mM sodium pyruvate, 100 unit/mL penicillin, 100 µg/mL streptomycin (Gibco: catalog No. 15140-122)) (hereinafter, referred to as "DMEM-10F"). Collagen-coated flasks (IWAKI: catalog Nos. 4100-010 and 4160-010) were used for culture. 293T cells were seeded on a collagen-coated 10-cm dish (IWAKI: catalog No. 4020-010) to 2.5 x 10⁶ cells/dish, and then cultured overnight. Opti-MEM (Gibco: catalog No. 11058), FuGENE6 (Roche: catalog No. 11814443001), and 3 types of construct (HCV envelope protein expression vector, Gag-Pol 5349, and Luc126) were mixed in the following amounts. Specifically, 500 µL of Opti-MEM, 21.6 µL of FuGENE6, 1 µg of HCV envelope protein expression vector, 3.1 µg of Gag-Pol 5349, and 3.1 µg of Luc126 (or mixed with the same composition ratio) were mixed, and then incubation was performed at room temperature for 15 minutes. The medium for 293T cells was exchanged with Opti-MEM (7.5 mL), a DNA complex was added to the medium, and then incubation was performed at 37°C and 5%CO₂ for 6 hours. After completion of the reaction, washing was performed once with PBS, 8 mL of DMEM-10F was added, and then incubation was performed at 37°C and 5%CO₂ for 48 hours. After completion of the culture, supernatants were collected and then filtered with a 0.45-µm filter, so that HCVpp solutions were obtained. HCVpp (1 mL) was dispensed and then stored at -80°C.

The thus obtained, pseudo HCV particles having structural proteins of the J6CF strain of genotype 2a were referred to as "J6CF HCVpp", pseudo HCV particles having structural proteins of the H77 strain of genotype 1a were referred to as "H77 HCVpp", and pseudo HCV particles having structural proteins of the TH strain of genotype 1b were referred to as "TH HCVpp".

### 2) Measurement of activity of inhibiting HCV infection

The serum samples obtained via blood collection from mice immunized via administration of each vaccine composition as in "1. Administration of vaccine composition" above were subjected to measurement of the activity of inhibiting HCV infection by the following method.

### (A) Activity of inhibiting HCV infection using HCVpp virus

Huh7.5.1 cells were seeded on 96-well plates (coated with poly-D-lysin) at 1 x 10⁴ cells/well, and then cultured overnight. A J6CF HCVpp virus solution (culture supernatant) obtained by the step 1) above was mixed with mouse serum in an amount equivalent thereto, and then incubated at room temperature for 30 minutes. Mouse serum was diluted 50-fold with DMEM medium (DMEM containing 10% FCS (Invitrogen), 1% MEM nonessential amino acid solution (Invitrogen), 10 mM HEPES-Tris (pH 7.3), and 1 mM sodium pyruvate) and then used (the final dilution rate for mouse serum: 100 fold). Medium for cells was discarded, and then mouse serum was added for incubation. The thus incubated virus solution was added at 50 µL/well and then incubated at 37°C for 3 hours. The virus solution was discarded, wells were washed once with PBS at 100 µL/well, DMEM medium was added at 200 µL/well, and then incubation was performed at 37°C for 72 hours. After medium was discarded, 25 µL/well DMEM medium and 25 µL/well Steady-Glo (Promega: catalog No. E2520) were added. Cells were lysed according to instructions included with Steady-Glo. The cell lysis solution (40 µL/well) was transferred to a white 96-well plate (Sumitomo Bakelite Co., Ltd.: catalog No. MS-8496W) and then luminescence intensity was measured using ARVO X4 (PerkinElmer).

Furthermore, to confirm whether or not serum samples derived from mice immunized with J6/JFH1-HCV particles having structural proteins of genotype 2a exhibited the activity of inhibiting HCV infection against HCV having structural proteins of genotype 1a and 1b, the activity of inhibiting HCV infection in mouse serum was measured by the method similar to the above using H77 HCVpp (pseudo HCV particles having structural proteins of genotype 1a) and TH HCVpp (pseudo HCV particles having structural proteins of genotype 1b).

### (B) Activity of inhibiting HCV infection using HCVcc virus

Huh7.5.1 cells were seeded on 96-well plates (coated with poly-D-lysin) at 1 x 10⁴ cells/well, and then cultured overnight. A J6/JFH1-HCV particle solution (a concentrated solution of the culture supernatant containing HCV particles; hereinafter, referred to as "J6/JFH1 HCVcc") obtained by the step 1) in Example 2 above was used as an HCVcc virus solution. The solution was mixed with mouse serum in an amount equivalent thereto, and then incubated at room temperature for 30 minutes. Mouse serum was diluted 50-fold with DMEM medium (DMEM containing 10% FCS (Invitrogen), 1% MEM nonessential amino acid solution (Invitrogen), 10 mM HEPES-Tris (pH 7.3), and 1 mM sodium pyruvate) and then used (the final dilution rate for mouse serum: 100 fold). Medium for cells was discarded, and then mouse serum was added for incubation. The thus incubated virus solution was added at 50 µL/well and then incubated at 37°C for 3 hours. The virus solution was discarded, wells were washed once with PBS at 100 µL/well, DMEM medium was added at 200 µL/well, and then incubation was performed at 37°C for 72 hours. After the culture supernatant was removed, the plate was soaked in ice-cold methanol to fix the cells. Thereafter, methanol was removed via air drying, and then Block Ace (registered trademark) (Dainippon Pharmaceutical Co., Ltd.) containing 0.3% Triton (registered trademark)-X 100 (GE Healthcare) was added at 100 µL/well, thereby performing blocking overnight at 4°C. Washing was performed twice with PBS 150 µL/well, HRP-labeled anti-HCV-core antibody diluted 100-fold (Ortho Clinical Diagnostics) was added at 50 µL/well, followed by 1 hour of reaction. Washing was then performed 4 times with PBS 150 µL/well, a QuantaBlu (registered trademark) (PIERCE) reaction solution was added at 50 µL/well, the resultant was left to stand at room temperature for 30 minutes, and then a QuantaBlu stop solution was added at 50 µL/well to stop the reaction. The resultant (20 µL/well) was transferred to a black 384-well plate (Corning: catalog No. 3676) and then fluorescence intensity was measured using ARVO X4 (PerkinElmer).

Furthermore, to confirm whether or not serum samples derived from mice immunized with J6/JFH1-HCV particles having structural proteins of genotype 2a had the activity of inhibiting HCV infection against HCVcc having structural proteins of genotype 1b, the activity of inhibiting HCV infection in serum was measured by the method similar to the above using a TH/JFH1-HCV particle solution (HCVcc particles having structural proteins of genotype 1b) (hereinafter, referred to as "TH/JFH1 HCVcc"). In addition, TH/JFH1 HCVcc was prepared by the method described in WO2009/131203.

### 4. Evaluation of immune-response-inducing capacity of vaccine composition containing J6E2Fc protein as antigen

### 1) Immune-response-inducing capacity of vaccine composition containing J6E2Fc protein as antigen

Fig. 1 shows the results of measuring antibody titers ("2. Measurement of antibody titers against E1 protein and E2 protein" above) against the E2 protein of mice to which the vaccine composition containing the J6E2Fc protein was administered as in 1) of "1. Administration of vaccine composition" above. In Fig. 1, "control" denotes the serum of a normal mouse to which no vaccine composition was administered, "5 µg or 20 µg" denotes the amounts of the J6E2Fc protein administered, and "x 1000, x 3000, x 10000" denotes dilution rates for mouse serum. As shown in Fig. 1, it was confirmed that Alum+Mod87s exhibited stronger activity of inducing the antibody against the E2 protein compared with Alum or Mod87s alone.

Also, from among the results of measuring neutralization titer (inhibition (%) of infection) ("3. Measurement of neutralization titer" above) for mice to which the vaccine composition containing the J6E2Fc protein was administered as in 1) of "1. Administration of vaccine composition," Fig. 2 shows the results for mice to which the vaccine composition containing 20 µg of the J6E2Fc protein (which exhibited the highest antibody titer against the E2 protein) and Alum+Mod87s as an adjuvant was administered. As shown in Fig. 2, the serum of the immunized mice merely exhibited the inhibition (12%) of HCV infection against J6CF HCVpp.

Therefore, it was demonstrated that antibody titer against the E2 protein of the vaccine composition containing the J6E2Fc protein as an antigen is increased , even when Alum+Mod87s had been used as an adjuvant, but no activity of inhibiting HCV infection is observed.

### 2) Immune-response-inducing capacity of vaccine composition containing inactivated J6/JFH1-HCV particle as antigen

Fig. 3 and Fig. 4 show the results of measuring antibody titer against the E1 protein or the E2 protein ("2. Measurement of antibody titers against E1 protein and E2 protein" above) for mice to which the vaccine composition containing inactivated J6/JFH1-HCV was administered as in 2) of "1. Administration of vaccine composition" above. As shown in Fig. 3, antibody titer of the vaccine composition containing polyI:C alone, Alum+Mod87s, Alum+poly I:C, or Mod87s+poly I:C against the E1 protein is increased compared with that of the vaccine composition containing Alum alone or Mod87s alone. Also, as shown in Fig. 4, antibody titer of the vaccine composition containing polyI:C alone, Alum+Mod87s, Alum+poly I:C, or Mod87s+poly I:C against the E2 protein is increased compared with that of the vaccine composition containing Alum alone or Mod87s alone.

Furthermore, Fig. 5 shows the results of measuring neutralization titer (inhibition (%) of infection) ("3. Measurement of neutralization titer" above) for mice to which the vaccine composition containing inactivated J6/JFH1-HCV was administered as in 2) of "1. Administration of vaccine composition" above. As shown in Fig. 5, the vaccine composition containing a combination of Alum and Mod87s as an adjuvant exhibited the activity (60%) of inhibiting HCV infection against J6CF HCVpp. On the other hand, in the case of the other vaccine compositions containing other adjuvants, specifically, the activity (47%) of inhibiting HCV infection was merely exhibited in mice to which the vaccine composition containing Alum alone as an adjuvant (exhibited the highest activity of inhibiting HCV infection) had been administered.

Fig. 6 (H77 HCVpp (pseudo HCV particles having structural proteins of genotype 1a)) and Fig. 7 (TH HCVpp (pseudo HCV particles having structural proteins of genotype 1b)) show the results of examining the capacity of inhibiting infection with HCV of genotype other than genotype 2a used for immunization for the serum samples of mice immunized with inactivated J6/JFH1-HCV as in 2) of "3. Measurement of neutralization titer" above. The results of measuring the activity of inhibiting HCV infection using H77 HCVpp of genotype 1a were: the activity (84%) of inhibiting HCV infection of the vaccine composition containing Alum+Mod87s as an adjuvant; and the activity (70%) of inhibiting HCV infection of the vaccine composition containing Alum alone as an adjuvant (Fig. 6). On the other hand, the results of measuring the activity of inhibiting HCV infection using TH HCVpp of genotype 1b were: the activity (72%) of inhibiting HCV infection of the vaccine composition containing Alum+Mod87s as an adjuvant; and the activity (55%) of inhibiting HCV infection of the vaccine composition containing Alum alone as an adjuvant (Fig. 7).

Fig. 8 shows the results of measuring neutralization titers ("3. Measurement of neutralization titers" above) using J6/JFH1 HCVcc of genotype 2a for the serum samples of mice to which the vaccine composition containing inactivated J6/JFH1-HCV was administered as in 2) of "1. Administration of vaccine composition" above. As shown in Fig. 8, the vaccine composition containing Alum+Mod87s as an adjuvant exhibited the activity (56%) of inhibiting HCV infection. On the other hand, the vaccine compositions containing other adjuvants merely exhibited inhibitory activity (26%) in mice to which the vaccine compositions containing Mod87s alone and poly I:C alone as adjuvants (exhibited the highest activity of inhibiting HCV infection) had been administered.

Moreover, Fig. 9 shows the results of examining the infection-inhibiting capacity of HCVcc of genotype other than genotype 2a used for immunization for the serum samples of mice to which the vaccine composition containing inactivated J6/JFH1-HCV was administered as in 2) of "3. Measurement of neutralization titer" above. As shown in Fig. 9, the results of measuring the activity of inhibiting HCV infection using TH/JFH1 HCVcc of genotype 1b were: the activity (42%) of inhibiting HCV infection in the case of the vaccine composition containing Alum+Mod87s as an adjuvant; and 27% in the case of vaccine compositions containing other adjuvants, and specifically in the case of the vaccine composition containing as an adjuvant Mod87s+poly I:C having the highest activity of inhibiting HCV infection.

These results revealed that the Alum+Mod87s adjuvant induces predominantly antibodies exhibiting the activity of inhibiting HCV infection. Moreover, these results demonstrate that among antibodies to be induced by immunization with HCV of genotype 2a, not only an antibody inhibiting infection with HCV of genotype 2a, but also an antibody inhibiting infection with HCV of genotype 1a and 1b is present.

### Sequence listing free text

SEQ ID NO: 1: JFH1
SEQ ID NO: 2: synthetic DNA J6/JFH1
SEQ ID NO: 3: synthetic DNA TH/JFH1
SEQ ID NO: 4: synthetic oligonucleotide Mod87
SEQ ID NO: 5: synthetic oligonucleotide Mod87s. 1st to 7th and 20th bases are phosphorothioated guanines.

SEQ ID NO: 6: primer J6E2Fc-s
SEQ ID NO: 7: primer J6E2Fc-as
SEQ ID NO: 8: primer J6E2dTM-as

## Claims

1. A hepatitis C virus vaccine composition, comprising:
inactivated viral particles obtained by inactivating infectious hepatitis C virus particles prepared from hepatitis C virus genome that contains sequences encoding NS3 protein, NS4A protein, NS4B protein, NS5A protein and NS5B protein derived from hepatitis C virus JFH1 strain;
an oligonucleotide containing unmethylated CpG, according to SEQ ID NO: 5 in the sequence listing; and
aluminium hydroxide.

2. The hepatitis C virus vaccine composition according to claim 1, wherein the hepatitis C virus genome contains sequences encoding core protein, E1 protein, E2 protein and p7 protein derived from hepatitis C virus J6CF strain.

3. The hepatitis C virus vaccine composition according to claim 1 or 2, wherein the hepatitis C virus genome contains:
a sequence that encodes 16 amino acid residues from the N-terminal amino acid residue of NS2 protein derived from hepatitis C virus J6CF strain; and
a sequence that encodes a portion ranging from the 17th amino acid residue from the N terminus to the C-terminal amino acid residue of NS2 protein derived from hepatitis C virus JFH1 strain.

4. A hepatitis C virus vaccine composition, comprising:
inactivated viral particles obtained by inactivating infectious hepatitis C virus particles containing NS3 protein, NS4A protein, NS4B protein, NS5A protein and NS5B protein derived from hepatitis C virus JFH1 strain;
an oligonucleotide containing unmethylated CpG, according to SEQ ID NO: 5 in the sequence listing; and
aluminium hydroxide.

5. The hepatitis C virus vaccine composition according to claim 3, wherein the infectious hepatitis C virus contains core protein, E1 protein, E2 protein and p7 protein derived from the hepatitis C virus J6CF strain.

6. The hepatitis C virus vaccine composition according to claim 4 or 5, wherein the infectious hepatitis C virus is **characterized in that** the NS2 protein is a chimeric protein, 16 amino acid residues from the N-terminal amino acid residue of the NS2 protein are derived from the J6CF strain, and a portion ranging from the 17th amino acid residue from the N-terminus to the C-terminal amino acid residue of the NS2 protein is derived from the JFH1 strain.


### Sequence Listing

SEQUENCE LISTING

<110> TORAY INDUSTRIES, INC.
JAPAN AS REPRESENTED BY DIRECTOR GENERAL OF NATIONAL INSTITUTE OF
INFECTIOUS DISEASES
TOKYO METROPOLITAN ORGANIZATION FOR MEDICAL RESEARCH

<120> Hepatitis C virus vaccine composition

<130> PH-4532-PCT

<150> JP 2009-228145
<151> 2009-09-30

<160> 10

<170> PatentIn version 3.4

<210> 1
<211> 9678
<212> DNA
<213> Hepatitis C virus

<220>
<223> JFH1

<400> 1
acctgcccct aataggggcg acactccgcc atgaatcact cccctgtgag gaactactgt 60

cttcacgcag aaagcgccta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

cgcttgcgag tgccccggga ggtctcgtag accgtgcacc atgagcacaa atcctaaacc 360

tcaaagaaaa accaaaagaa acaccaaccg tcgcccagaa gacgttaagt tcccgggcgg 420

cggccagatc gttggcggag tatacttgtt gccgcgcagg ggccccaggt tgggtgtgcg 480

cacgacaagg aaaacttcgg agcggtccca gccacgtggg agacgccagc ccatccccaa 540

agatcggcgc tccactggca aggcctgggg aaaaccaggt cgcccctggc ccctatatgg 600

gaatgaggga ctcggctggg caggatggct cctgtccccc cgaggctctc gcccctcctg 660

gggccccact gacccccggc ataggtcgcg caacgtgggt aaagtcatcg acaccctaac 720

gtgtggcttt gccgacctca tggggtacat ccccgtcgta ggcgccccgc ttagtggcgc 780

cgccagagct gtcgcgcacg gcgtgagagt cctggaggac ggggttaatt atgcaacagg 840

gaacctaccc ggtttcccct tttctatctt cttgctggcc ctgttgtcct gcatcaccgt 900

tccggtctct gctgcccagg tgaagaatac cagtagcagc tacatggtga ccaatgactg 960

ctccaatgac agcatcactt ggcagctcga ggctgcggtt ctccacgtcc ccgggtgcgt 1020

cccgtgcgag agagtgggga atacgtcacg gtgttgggtg ccagtctcgc caaacatggc 1080

tgtgcggcag cccggtgccc tcacgcaggg tctgcggacg cacatcgata tggttgtgat 1140

gtccgccacc ttctgctctg ctctctacgt gggggacctc tgtggcgggg tgatgctcgc 1200

ggcccaggtg ttcatcgtct cgccgcagta ccactggttt gtgcaagaat gcaattgctc 1260

catctaccct ggcaccatca ctggacaccg catggcatgg gacatgatga tgaactggtc 1320

gcccacggcc accatgatcc tggcgtacgt gatgcgcgtc cccgaggtca tcatagacat 1380

cgttagcggg gctcactggg gcgtcatgtt cggcttggcc tacttctcta tgcagggagc 1440

gtgggcgaag gtcattgtca tccttctgct ggccgctggg gtggacgcgg gcaccaccac 1500

cgttggaggc gctgttgcac gttccaccaa cgtgattgcc ggcgtgttca gccatggccc 1560

tcagcagaac attcagctca ttaacaccaa cggcagttgg cacatcaacc gtactgcctt 1620

gaattgcaat gactccttga acaccggctt tctcgcggcc ttgttctaca ccaaccgctt 1680

taactcgtca gggtgtccag ggcgcctgtc cgcctgccgc aacatcgagg ctttccggat 1740

agggtggggc accctacagt acgaggataa tgtcaccaat ccagaggata tgaggccgta 1800

ctgctggcac taccccccaa agccgtgtgg cgtagtcccc gcgaggtctg tgtgtggccc 1860

agtgtactgt ttcaccccca gcccggtagt agtgggcacg accgacagac gtggagtgcc 1920

cacctacaca tggggagaga atgagacaga tgtcttccta ctgaacagca cccgaccgcc 1980

gcagggctca tggttcggct gcacgtggat gaactccact ggtttcacca agacttgtgg 2040

cgcgccacct tgccgcacca gagctgactt caacgccagc acggacttgt tgtgccctac 2100

ggattgtttt aggaagcatc ctgatgccac ttatattaag tgtggttctg ggccctggct 2160

cacaccaaag tgcctggtcc actaccctta cagactctgg cattacccct gcacagtcaa 2220

ttttaccatc ttcaagataa gaatgtatgt agggggggtt gagcacaggc tcacggccgc 2280

atgcaacttc actcgtgggg atcgctgcga cttggaggac agggacagga gtcagctgtc 2340

tcctctgttg cactctacca cggaatgggc catcctgccc tgcacctact cagacttacc 2400

cgctttgtca actggtcttc tccaccttca ccagaacatc gtggacgtac aatacatgta 2460

tggcctctca cctgctatca caaaatacgt cgttcgatgg gagtgggtgg tactcttatt 2520

cctgctctta gcggacgcca gagtctgcgc ctgcttgtgg atgctcatct tgttgggcca 2580

ggccgaagca gcattggaga agttggtcgt cttgcacgct gcgagtgcgg ctaactgcca 2640

tggcctccta tattttgcca tcttcttcgt ggcagcttgg cacatcaggg gtcgggtggt 2700

ccccttgacc acctattgcc tcactggcct atggcccttc tgcctactgc tcatggcact 2760

gccccggcag gcttatgcct atgacgcacc tgtgcacgga cagataggcg tgggtttgtt 2820

gatattgatc accctcttca cactcacccc ggggtataag accctcctcg gccagtgtct 2880

gtggtggttg tgctatctcc tgaccctggg ggaagccatg attcaggagt gggtaccacc 2940

catgcaggtg cgcggcggcc gcgatggcat cgcgtgggcc gtcactatat tctgcccggg 3000

tgtggtgttt gacattacca aatggctttt ggcgttgctt gggcctgctt acctcttaag 3060

ggccgctttg acacatgtgc cgtacttcgt cagagctcac gctctgataa gggtatgcgc 3120

tttggtgaag cagctcgcgg ggggtaggta tgttcaggtg gcgctattgg cccttggcag 3180

gtggactggc acctacatct atgaccacct cacacctatg tcggactggg ccgctagcgg 3240

cctgcgcgac ttagcggtcg ccgtggaacc catcatcttc agtccgatgg agaagaaggt 3300

catcgtctgg ggagcggaga cggctgcatg tggggacatt ctacatggac ttcccgtgtc 3360

cgcccgactc ggccaggaga tcctcctcgg cccagctgat ggctacacct ccaaggggtg 3420

gaagctcctt gctcccatca ctgcttatgc ccagcaaaca cgaggcctcc tgggcgccat 3480

agtggtgagt atgacggggc gtgacaggac agaacaggcc ggggaagtcc aaatcctgtc 3540

cacagtctct cagtccttcc tcggaacaac catctcgggg gttttgtgga ctgtttacca 3600

cggagctggc aacaagactc tagccggctt acggggtccg gtcacgcaga tgtactcgag 3660

tgctgagggg gacttggtag gctggcccag cccccctggg accaagtctt tggagccgtg 3720

caagtgtgga gccgtcgacc tatatctggt cacgcggaac gctgatgtca tcccggctcg 3780

gagacgcggg gacaagcggg gagcattgct ctccccgaga cccatttcga ccttgaaggg 3840

gtcctcgggg gggccggtgc tctgccctag gggccacgtc gttgggctct tccgagcagc 3900

tgtgtgctct cggggcgtgg ccaaatccat cgatttcatc cccgttgaga cactcgacgt 3960

tgttacaagg tctcccactt tcagtgacaa cagcacgcca ccggctgtgc cccagaccta 4020

tcaggtcggg tacttgcatg ctccaactgg cagtggaaag agcaccaagg tccctgtcgc 4080

gtatgccgcc caggggtaca aagtactagt gcttaacccc tcggtagctg ccaccctggg 4140

gtttggggcg tacctatcca aggcacatgg catcaatccc aacattagga ctggagtcag 4200

gaccgtgatg accggggagg ccatcacgta ctccacatat ggcaaatttc tcgccgatgg 4260

gggctgcgct agcggcgcct atgacatcat catatgcgat gaatgccacg ctgtggatgc 4320

tacctccatt ctcggcatcg gaacggtcct tgatcaagca gagacagccg gggtcagact 4380

aactgtgctg gctacggcca caccccccgg gtcagtgaca accccccatc ccgatataga 4440

agaggtaggc ctcgggcggg agggtgagat ccccttctat gggagggcga ttcccctatc 4500

ctgcatcaag ggagggagac acctgatttt ctgccactca aagaaaaagt gtgacgagct 4560

cgcggcggcc cttcggggca tgggcttgaa tgccgtggca tactatagag ggttggacgt 4620

ctccataata ccagctcagg gagatgtggt ggtcgtcgcc accgacgccc tcatgacggg 4680

gtacactgga gactttgact ccgtgatcga ctgcaatgta gcggtcaccc aagctgtcga 4740

cttcagcctg gaccccacct tcactataac cacacagact gtcccacaag acgctgtctc 4800

acgcagtcag cgccgcgggc gcacaggtag aggaagacag ggcacttata ggtatgtttc 4860

cactggtgaa cgagcctcag gaatgtttga cagtgtagtg ctttgtgagt gctacgacgc 4920

aggggctgcg tggtacgatc tcacaccagc ggagaccacc gtcaggctta gagcgtattt 4980

caacacgccc ggcctacccg tgtgtcaaga ccatcttgaa ttttgggagg cagttttcac 5040

cggcctcaca cacatagacg cccacttcct ctcccaaaca aagcaagcgg gggagaactt 5100

cgcgtaccta gtagcctacc aagctacggt gtgcgccaga gccaaggccc ctcccccgtc 5160

ctgggacgcc atgtggaagt gcctggcccg actcaagcct acgcttgcgg gccccacacc 5220

tctcctgtac cgtttgggcc ctattaccaa tgaggtcacc ctcacacacc ctgggacgaa 5280

gtacatcgcc acatgcatgc aagctgacct tgaggtcatg accagcacgt gggtcctagc 5340

tggaggagtc ctggcagccg tcgccgcata ttgcctggcg actggatgcg tttccatcat 5400

cggccgcttg cacgtcaacc agcgagtcgt cgttgcgccg gataaggagg tcctgtatga 5460

ggcttttgat gagatggagg aatgcgcctc tagggcggct ctcatcgaag aggggcagcg 5520

gatagccgag atgttgaagt ccaagatcca aggcttgctg cagcaggcct ctaagcaggc 5580

ccaggacata caacccgcta tgcaggcttc atggcccaaa gtggaacaat tttgggccag 5640

acacatgtgg aacttcatta gcggcatcca atacctcgca ggattgtcaa cactgccagg 5700

gaaccccgcg gtggcttcca tgatggcatt cagtgccgcc ctcaccagtc cgttgtcgac 5760

cagtaccacc atccttctca acatcatggg aggctggtta gcgtcccaga tcgcaccacc 5820

cgcgggggcc accggctttg tcgtcagtgg cctggtgggg gctgccgtgg gcagcatagg 5880

cctgggtaag gtgctggtgg acatcctggc aggatatggt gcgggcattt cgggggccct 5940

cgtcgcattc aagatcatgt ctggcgagaa gccctctatg gaagatgtca tcaatctact 6000

gcctgggatc ctgtctccgg gagccctggt ggtgggggtc atctgcgcgg ccattctgcg 6060

ccgccacgtg ggaccggggg agggcgcggt ccaatggatg aacaggctta ttgcctttgc 6120

ttccagagga aaccacgtcg cccctactca ctacgtgacg gagtcggatg cgtcgcagcg 6180

tgtgacccaa ctacttggct ctcttactat aaccagccta ctcagaagac tccacaattg 6240

gataactgag gactgcccca tcccatgctc cggatcctgg ctccgcgacg tgtgggactg 6300

ggtttgcacc atcttgacag acttcaaaaa ttggctgacc tctaaattgt tccccaagct 6360

gcccggcctc cccttcatct cttgtcaaaa ggggtacaag ggtgtgtggg ccggcactgg 6420

catcatgacc acgcgctgcc cttgcggcgc caacatctct ggcaatgtcc gcctgggctc 6480

tatgaggatc acagggccta aaacctgcat gaacacctgg caggggacct ttcctatcaa 6540

ttgctacacg gagggccagt gcgcgccgaa accccccacg aactacaaga ccgccatctg 6600

gagggtggcg gcctcggagt acgcggaggt gacgcagcat gggtcgtact cctatgtaac 6660

aggactgacc actgacaatc tgaaaattcc ttgccaacta ccttctccag agtttttctc 6720

ctgggtggac ggtgtgcaga tccataggtt tgcacccaca ccaaagccgt ttttccggga 6780

tgaggtctcg ttctgcgttg ggcttaattc ctatgctgtc gggtcccagc ttccctgtga 6840

acctgagccc gacgcagacg tattgaggtc catgctaaca gatccgcccc acatcacggc 6900

ggagactgcg gcgcggcgct tggcacgggg atcacctcca tctgaggcga gctcctcagt 6960

gagccagcta tcagcaccgt cgctgcgggc cacctgcacc acccacagca acacctatga 7020

cgtggacatg gtcgatgcca acctgctcat ggagggcggt gtggctcaga cagagcctga 7080

gtccagggtg cccgttctgg actttctcga gccaatggcc gaggaagaga gcgaccttga 7140

gccctcaata ccatcggagt gcatgctccc caggagcggg tttccacggg ccttaccggc 7200

ttgggcacgg cctgactaca acccgccgct cgtggaatcg tggaggaggc cagattacca 7260

accgcccacc gttgctggtt gtgctctccc cccccccaag aaggccccga cgcctccccc 7320

aaggagacgc cggacagtgg gtctgagcga gagcaccata tcagaagccc tccagcaact 7380

ggccatcaag acctttggcc agcccccctc gagcggtgat gcaggctcgt ccacgggggc 7440

gggcgccgcc gaatccggcg gtccgacgtc ccctggtgag ccggccccct cagagacagg 7500

ttccgcctcc tctatgcccc ccctcgaggg ggagcctgga gatccggacc tggagtctga 7560

tcaggtagag cttcaacctc ccccccaggg ggggggggta gctcccggtt cgggctcggg 7620

gtcttggtct acttgctccg aggaggacga taccaccgtg tgctgctcca tgtcatactc 7680

ctggaccggg gctctaataa ctccctgtag ccccgaagag gaaaagttgc caatcaaccc 7740

tttgagtaac tcgctgttgc gataccataa caaggtgtac tgtacaacat caaagagcgc 7800

ctcacagagg gctaaaaagg taacttttga caggacgcaa gtgctcgacg cccattatga 7860

ctcagtctta aaggacatca agctagcggc ttccaaggtc agcgcaaggc tcctcacctt 7920

ggaggaggcg tgccagttga ctccacccca ttctgcaaga tccaagtatg gattcggggc 7980

caaggaggtc cgcagcttgt ccgggagggc cgttaaccac atcaagtccg tgtggaagga 8040

cctcctggaa gacccacaaa caccaattcc cacaaccatc atggccaaaa atgaggtgtt 8100

ctgcgtggac cccgccaagg ggggtaagaa accagctcgc ctcatcgttt accctgacct 8160

cggcgtccgg gtctgcgaga aaatggccct ctatgacatt acacaaaagc ttcctcaggc 8220

ggtaatggga gcttcctatg gcttccagta ctcccctgcc caacgggtgg agtatctctt 8280

gaaagcatgg gcggaaaaga aggaccccat gggtttttcg tatgataccc gatgcttcga 8340

ctcaaccgtc actgagagag acatcaggac cgaggagtcc atataccagg cctgctccct 8400

gcccgaggag gcccgcactg ccatacactc gctgactgag agactttacg taggagggcc 8460

catgttcaac agcaagggtc aaacctgcgg ttacagacgt tgccgcgcca gcggggtgct 8520

aaccactagc atgggtaaca ccatcacatg ctatgtgaaa gccctagcgg cctgcaaggc 8580

tgcggggata gttgcgccca caatgctggt atgcggcgat gacctagtag tcatctcaga 8640

aagccagggg actgaggagg acgagcggaa cctgagagcc ttcacggagg ccatgaccag 8700

gtactctgcc cctcctggtg atccccccag accggaatat gacctggagc taataacatc 8760

ctgttcctca aatgtgtctg tggcgttggg cccgcggggc cgccgcagat actacctgac 8820

cagagaccca accactccac tcgcccgggc tgcctgggaa acagttagac actcccctat 8880

caattcatgg ctgggaaaca tcatccagta tgctccaacc atatgggttc gcatggtcct 8940

aatgacacac ttcttctcca ttctcatggt ccaagacacc ctggaccaga acctcaactt 9000

tgagatgtat ggatcagtat actccgtgaa tcctttggac cttccagcca taattgagag 9060

gttacacggg cttgacgcct tttctatgca cacatactct caccacgaac tgacgcgggt 9120

ggcttcagcc ctcagaaaac ttggggcgcc acccctcagg gtgtggaaga gtcgggctcg 9180

cgcagtcagg gcgtccctca tctcccgtgg agggaaagcg gccgtttgcg gccgatatct 9240

cttcaattgg gcggtgaaga ccaagctcaa actcactcca ttgccggagg cgcgcctact 9300

ggacttatcc agttggttca ccgtcggcgc cggcgggggc gacatttttc acagcgtgtc 9360

gcgcgcccga ccccgctcat tactcttcgg cctactccta cttttcgtag gggtaggcct 9420

cttcctactc cccgctcggt agagcggcac acactaggta cactccatag ctaactgttc 9480

cttttttttt tttttttttt tttttttttt tttttttttt ttttcttttt tttttttttc 9540

cctctttctt cccttctcat cttattctac tttctttctt ggtggctcca tcttagccct 9600

agtcacggct agctgtgaaa ggtccgtgag ccgcatgact gcagagagtg ccgtaactgg 9660

tctctctgca gatcatgt 9678

<210> 2
<211> 9683
<212> DNA
<213> Artificial

<220>
<223> synthetic DNA J6/JFH1

<400> 2
acccgcccct aataggggcg acactccgcc atgaatcact cccctgtgag gaactactgt 60

cttcacgcag aaagcgtcta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

tgcttgcgag tgccccggga ggtctcgtag accgtgcacc atgagcacaa atcctaaacc 360

tcaaagaaaa accaaaagaa acaccaaccg tcgcccacaa gacgttaagt ttccgggcgg 420

cggccagatc gttggcggag tatacttgtt gccgcgcagg ggccccaggt tgggtgtgcg 480

cgcgacaagg aagacttcgg agcggtccca gccacgtgga aggcgccagc ccatccctaa 540

agatcggcgc tccactggca aatcctgggg aaaaccagga tacccctggc ccctatacgg 600

gaatgaggga ctcggctggg caggatggct cctgtccccc cgaggttccc gtccctcttg 660

gggccccaat gacccccggc ataggtcgcg caacgtgggt aaggtcatcg ataccctaac 720

gtgcggcttt gccgacctca tggggtacat ccctgtcgtg ggcgccccgc tcggcggcgt 780

cgccagagct ctcgcgcatg gcgtgagagt cctggaggac ggggttaatt ttgcaacagg 840

gaacttaccc ggttgctcct tttctatctt cttgctggcc ctgctgtcct gcatcaccac 900

cccggtctcc gctgccgaag tgaagaacat cagtaccggc tacatggtga ctaacgactg 960

caccaatgac agcattacct ggcagctcca ggctgctgtc ctccacgtcc ccgggtgcgt 1020

cccgtgcgag aaagtgggga atgcatctca gtgctggata ccggtctcac cgaatgtggc 1080

cgtgcagcgg cccggcgccc tcacgcaggg cttgcggacg cacatcgaca tggttgtgat 1140

gtccgccacg ctctgctctg ccctctacgt gggggacctc tgcggtgggg tgatgctcgc 1200

agcccaaatg ttcattgtct cgccgcagca ccactggttt gtccaagact gcaattgctc 1260

catctaccct ggtaccatca ctggacaccg catggcatgg gacatgatga tgaactggtc 1320

gcccacggct accatgatct tggcgtacgc gatgcgtgtc cccgaggtca ttatagacat 1380

cattagcggg gctcattggg gcgtcatgtt cggcttggcc tacttctcta tgcagggagc 1440

gtgggcgaaa gtcgttgtca tccttctgtt ggccgccggg gtggacgcgc gcacccatac 1500

tgttgggggt tctgccgcgc agaccaccgg gcgcctcacc agcttatttg acatgggccc 1560

caggcagaaa atccagctcg ttaacaccaa tggcagctgg cacatcaacc gcaccgccct 1620

gaactgcaat gactccttgc acaccggctt tatcgcgtct ctgttctaca cccacagctt 1680

caactcgtca ggatgtcccg aacgcatgtc cgcctgccgc agtatcgagg ccttccgggt 1740

gggatggggc gccttgcaat atgaggataa tgtcaccaat ccagaggata tgagacccta 1800

ttgctggcac tacccaccaa ggcagtgtgg cgtggtctcc gcgaagactg tgtgtggccc 1860

agtgtactgt ttcaccccca gcccagtggt agtgggcacg accgacaggc ttggagcgcc 1920

cacttacacg tggggggaga atgagacaga tgtcttccta ttgaacagca ctcgaccacc 1980

gctggggtca tggttcggct gcacgtggat gaactcttct ggctacacca agacttgcgg 2040

cgcaccaccc tgccgtacta gagctgactt caacgccagc acggacctgt tgtgccccac 2100

ggactgtttt aggaagcatc ctgataccac ttacctcaaa tgcggctctg ggccctggct 2160

cacgccaagg tgcctgatcg actaccccta caggctctgg cattacccct gcacagttaa 2220

ctataccatc ttcaaaataa ggatgtatgt gggaggggtt gagcacaggc tcacggctgc 2280

atgcaatttc actcgtgggg atcgttgcaa cttggaggac agagacagaa gtcaactgtc 2340

tcctttgttg cactccacca cggaatgggc cattttacct tgctcttact cggacctgcc 2400

cgccttgtcg actggtcttc tccacctcca ccaaaacatc gtggacgtac aattcatgta 2460

tggcctatca cctgccctca caaaatacat cgtccgatgg gagtgggtaa tactcttatt 2520

cctgctctta gcggacgcca gggtttgcgc ctgcttatgg atgctcatct tgttgggcca 2580

ggccgaagca gcactagaga agctggtcat cttgcacgct gcgagcgcag ctagctgcaa 2640

tggcttccta tattttgtca tctttttcgt ggctgcttgg tacatcaagg gtcgggtagt 2700

ccccttagct acctattccc tcactggcct gtggtccttt agcctactgc tcctagcatt 2760

gccccaacag gcttatgctt atgacgcatc tgtgcatggc cagataggag cggctctgct 2820

ggtaatgatc accctcttca cactcacccc ggggtataag accctcctcg gccagtgtct 2880

gtggtggttg tgctatctcc tgaccctggg ggaagccatg attcaggagt gggtaccacc 2940

catgcaggtg cgcggcggcc gcgatggcat cgcgtgggcc gtcactatat tctgcccggg 3000

tgtggtgttt gacattacca aatggctttt ggcgttgctt gggcctgctt acctcttaag 3060

ggccgctttg acacatgtgc cgtacttcgt cagagctcac gctctgataa gggtatgcgc 3120

tttggtgaag cagctcgcgg ggggtaggta tgttcaggtg gcgctattgg cccttggcag 3180

gtggactggc acctacatct atgaccacct cacacctatg tcggactggg ccgctagcgg 3240

cctgcgcgac ttagcggtcg ccgtggaacc catcatcttc agtccgatgg agaagaaggt 3300

catcgtctgg ggagcggaga cggctgcatg tggggacatt ctacatggac ttcccgtgtc 3360

cgcccgactc ggccaggaga tcctcctcgg cccagctgat ggctacacct ccaaggggtg 3420

gaagctcctt gctcccatca ctgcttatgc ccagcaaaca cgaggcctcc tgggcgccat 3480

agtggtgagt atgacggggc gtgacaggac agaacaggcc ggggaagtcc aaatcctgtc 3540

cacagtctct cagtccttcc tcggaacaac catctcgggg gttttgtgga ctgtttacca 3600

cggagctggc aacaagactc tagccggctt acggggtccg gtcacgcaga tgtactcgag 3660

tgctgagggg gacttggtag gctggcccag cccccctggg accaagtctt tggagccgtg 3720

caagtgtgga gccgtcgacc tatatctggt cacgcggaac gctgatgtca tcccggctcg 3780

gagacgcggg gacaagcggg gagcattgct ctccccgaga cccatttcga ccttgaaggg 3840

gtcctcgggg gggccggtgc tctgccctag gggccacgtc gttgggctct tccgagcagc 3900

tgtgtgctct cggggcgtgg ccaaatccat cgatttcatc cccgttgaga cactcgacgt 3960

tgttacaagg tctcccactt tcagtgacaa cagcacgcca ccggctgtgc cccagaccta 4020

tcaggtcggg tacttgcatg ctccaactgg cagtggaaag agcaccaagg tccctgtcgc 4080

gtatgccgcc caggggtaca aagtactagt gcttaacccc tcggtagctg ccaccctggg 4140

gtttggggcg tacctatcca aggcacatgg catcaatccc aacattagga ctggagtcag 4200

gaccgtgatg accggggagg ccatcacgta ctccacatat ggcaaatttc tcgccgatgg 4260

gggctgcgct agcggcgcct atgacatcat catatgcgat gaatgccacg ctgtggatgc 4320

tacctccatt ctcggcatcg gaacggtcct tgatcaagca gagacagccg gggtcagact 4380

aactgtgctg gctacggcca caccccccgg gtcagtgaca accccccatc ccgatataga 4440

agaggtaggc ctcgggcggg agggtgagat ccccttctat gggagggcga ttcccctatc 4500

ctgcatcaag ggagggagac acctgatttt ctgccactca aagaaaaagt gtgacgagct 4560

cgcggcggcc cttcggggca tgggcttgaa tgccgtggca tactatagag ggttggacgt 4620

ctccataata ccagctcagg gagatgtggt ggtcgtcgcc accgacgccc tcatgacggg 4680

gtacactgga gactttgact ccgtgatcga ctgcaatgta gcggtcaccc aagctgtcga 4740

cttcagcctg gaccccacct tcactataac cacacagact gtcccacaag acgctgtctc 4800

acgcagtcag cgccgcgggc gcacaggtag aggaagacag ggcacttata ggtatgtttc 4860

cactggtgaa cgagcctcag gaatgtttga cagtgtagtg ctttgtgagt gctacgacgc 4920

aggggctgcg tggtacgatc tcacaccagc ggagaccacc gtcaggctta gagcgtattt 4980

caacacgccc ggcctacccg tgtgtcaaga ccatcttgaa ttttgggagg cagttttcac 5040

cggcctcaca cacatagacg cccacttcct ctcccaaaca aagcaagcgg gggagaactt 5100

cgcgtaccta gtagcctacc aagctacggt gtgcgccaga gccaaggccc ctcccccgtc 5160

ctgggacgcc atgtggaagt gcctggcccg actcaagcct acgcttgcgg gccccacacc 5220

tctcctgtac cgtttgggcc ctattaccaa tgaggtcacc ctcacacacc ctgggacgaa 5280

gtacatcgcc acatgcatgc aagctgacct tgaggtcatg accagcacgt gggtcctagc 5340

tggaggagtc ctggcagccg tcgccgcata ttgcctggcg actggatgcg tttccatcat 5400

cggccgcttg cacgtcaacc agcgagtcgt cgttgcgccg gataaggagg tcctgtatga 5460

ggcttttgat gagatggagg aatgcgcctc tagggcggct ctcatcgaag aggggcagcg 5520

gatagccgag atgttgaagt ccaagatcca aggcttgctg cagcaggcct ctaagcaggc 5580

ccaggacata caacccgcta tgcaggcttc atggcccaaa gtggaacaat tttgggccag 5640

acacatgtgg aacttcatta gcggcatcca atacctcgca ggattgtcaa cactgccagg 5700

gaaccccgcg gtggcttcca tgatggcatt cagtgccgcc ctcaccagtc cgttgtcgac 5760

cagtaccacc atccttctca acatcatggg aggctggtta gcgtcccaga tcgcaccacc 5820

cgcgggggcc accggctttg tcgtcagtgg cctggtgggg gctgccgtgg gcagcatagg 5880

cctgggtaag gtgctggtgg acatcctggc aggatatggt gcgggcattt cgggggccct 5940

cgtcgcattc aagatcatgt ctggcgagaa gccctctatg gaagatgtca tcaatctact 6000

gcctgggatc ctgtctccgg gagccctggt ggtgggggtc atctgcgcgg ccattctgcg 6060

ccgccacgtg ggaccggggg agggcgcggt ccaatggatg aacaggctta ttgcctttgc 6120

ttccagagga aaccacgtcg cccctactca ctacgtgacg gagtcggatg cgtcgcagcg 6180

tgtgacccaa ctacttggct ctcttactat aaccagccta ctcagaagac tccacaattg 6240

gataactgag gactgcccca tcccatgctc cggatcctgg ctccgcgacg tgtgggactg 6300

ggtttgcacc atcttgacag acttcaaaaa ttggctgacc tctaaattgt tccccaagct 6360

gcccggcctc cccttcatct cttgtcaaaa ggggtacaag ggtgtgtggg ccggcactgg 6420

catcatgacc acgcgctgcc cttgcggcgc caacatctct ggcaatgtcc gcctgggctc 6480

tatgaggatc acagggccta aaacctgcat gaacacctgg caggggacct ttcctatcaa 6540

ttgctacacg gagggccagt gcgcgccgaa accccccacg aactacaaga ccgccatctg 6600

gagggtggcg gcctcggagt acgcggaggt gacgcagcat gggtcgtact cctatgtaac 6660

aggactgacc actgacaatc tgaaaattcc ttgccaacta ccttctccag agtttttctc 6720

ctgggtggac ggtgtgcaga tccataggtt tgcacccaca ccaaagccgt ttttccggga 6780

tgaggtctcg ttctgcgttg ggcttaattc ctatgctgtc gggtcccagc ttccctgtga 6840

acctgagccc gacgcagacg tattgaggtc catgctaaca gatccgcccc acatcacggc 6900

ggagactgcg gcgcggcgct tggcacgggg atcacctcca tctgaggcga gctcctcagt 6960

gagccagcta tcagcaccgt cgctgcgggc cacctgcacc acccacagca acacctatga 7020

cgtggacatg gtcgatgcca acctgctcat ggagggcggt gtggctcaga cagagcctga 7080

gtccagggtg cccgttctgg actttctcga gccaatggcc gaggaagaga gcgaccttga 7140

gccctcaata ccatcggagt gcatgctccc caggagcggg tttccacggg ccttaccggc 7200

ttgggcacgg cctgactaca acccgccgct cgtggaatcg tggaggaggc cagattacca 7260

accgcccacc gttgctggtt gtgctctccc cccccccaag aaggccccga cgcctccccc 7320

aaggagacgc cggacagtgg gtctgagcga gagcaccata tcagaagccc tccagcaact 7380

ggccatcaag acctttggcc agcccccctc gagcggtgat gcaggctcgt ccacgggggc 7440

gggcgccgcc gaatccggcg gtccgacgtc ccctggtgag ccggccccct cagagacagg 7500

ttccgcctcc tctatgcccc ccctcgaggg ggagcctgga gatccggacc tggagtctga 7560

tcaggtagag cttcaacctc ccccccaggg ggggggggta gctcccggtt cgggctcggg 7620

gtcttggtct acttgctccg aggaggacga taccaccgtg tgctgctcca tgtcatactc 7680

ctggaccggg gctctaataa ctccctgtag ccccgaagag gaaaagttgc caatcaaccc 7740

tttgagtaac tcgctgttgc gataccataa caaggtgtac tgtacaacat caaagagcgc 7800

ctcacagagg gctaaaaagg taacttttga caggacgcaa gtgctcgacg cccattatga 7860

ctcagtctta aaggacatca agctagcggc ttccaaggtc agcgcaaggc tcctcacctt 7920

ggaggaggcg tgccagttga ctccacccca ttctgcaaga tccaagtatg gattcggggc 7980

caaggaggtc cgcagcttgt ccgggagggc cgttaaccac atcaagtccg tgtggaagga 8040

cctcctggaa gacccacaaa caccaattcc cacaaccatc atggccaaaa atgaggtgtt 8100

ctgcgtggac cccgccaagg ggggtaagaa accagctcgc ctcatcgttt accctgacct 8160

cggcgtccgg gtctgcgaga aaatggccct ctatgacatt acacaaaagc ttcctcaggc 8220

ggtaatggga gcttcctatg gcttccagta ctcccctgcc caacgggtgg agtatctctt 8280

gaaagcatgg gcggaaaaga aggaccccat gggtttttcg tatgataccc gatgcttcga 8340

ctcaaccgtc actgagagag acatcaggac cgaggagtcc atataccagg cctgctccct 8400

gcccgaggag gcccgcactg ccatacactc gctgactgag agactttacg taggagggcc 8460

catgttcaac agcaagggtc aaacctgcgg ttacagacgt tgccgcgcca gcggggtgct 8520

aaccactagc atgggtaaca ccatcacatg ctatgtgaaa gccctagcgg cctgcaaggc 8580

tgcggggata gttgcgccca caatgctggt atgcggcgat gacctagtag tcatctcaga 8640

aagccagggg actgaggagg acgagcggaa cctgagagcc ttcacggagg ccatgaccag 8700

gtactctgcc cctcctggtg atccccccag accggaatat gacctggagc taataacatc 8760

ctgttcctca aatgtgtctg tggcgttggg cccgcggggc cgccgcagat actacctgac 8820

cagagaccca accactccac tcgcccgggc tgcctgggaa acagttagac actcccctat 8880

caattcatgg ctgggaaaca tcatccagta tgctccaacc atatgggttc gcatggtcct 8940

aatgacacac ttcttctcca ttctcatggt ccaagacacc ctggaccaga acctcaactt 9000

tgagatgtat ggatcagtat actccgtgaa tcctttggac cttccagcca taattgagag 9060

gttacacggg cttgacgcct tttctatgca cacatactct caccacgaac tgacgcgggt 9120

ggcttcagcc ctcagaaaac ttggggcgcc acccctcagg gtgtggaaga gtcgggctcg 9180

cgcagtcagg gcgtccctca tctcccgtgg agggaaagcg gccgtttgcg gccgatatct 9240

cttcaattgg gcggtgaaga ccaagctcaa actcactcca ttgccggagg cgcgcctact 9300

ggacttatcc agttggttca ccgtcggcgc cggcgggggc gacatttttc acagcgtgtc 9360

gcgcgcccga ccccgctcat tactcttcgg cctactccta cttttcgtag gggtaggcct 9420

cttcctactc cccgctcggt agagcggcac acactaggta cactccatag ctaactgttc 9480

cttttttttt tttttttttt tttttttttt tttttttttt ttttcttttt tttttttttc 9540

cctctttctt cccttctcat cttattctac tttctttctt ggtggctcca tcttagccct 9600

agtcacggct agctgtgaaa ggtccgtgag ccgcatgact gcagagagtg ccgtaactgg 9660

tctctctgca gatcatgtct aga 9683

<210> 3
<211> 9669
<212> DNA
<213> Artificial

<220>
<223> synthetic DNA TH/JFH1

<400> 3
   acctgcccct aataggggcg acactccgcc atgaatcact cccctgtgag gaactactgt 60
   cttcacgcag aaagcgccta gccatggcgt tagtatgagt gtcgtacagc ctccaggccc 120
   ccccctcccg ggagagccat agtggtctgc ggaaccggtg agtacaccgg aattgccggg 180
   aagactgggt cctttcttgg ataaacccac tctatgcccg gccatttggg cgtgcccccg 240
   caagactgct agccgagtag cgttgggttg cgaaaggcct tgtggtactg cctgataggg 300
   cgcttgcgag tgccccggga ggtctcgtag accgtgcacc atgagcacga atcctaaacc 360
   tcaaagaaaa accaaacgta acaccaaccg ccgcccacag gacgtcaagt tcccgggcgg 420
   tggccagatc gttggtggag tttacctgtt gccgcgcagg ggccccaggt tgggtgtgcg 480
   cgcgactagg aagacttccg agcggtcgca acctcgtgga aggcgacaac ctatccccaa 540
   ggatcgccga cccgagggca gggcctgggc tcagcctggg tacccttggc ccctctatgg 600
   caacgagggc atggggtggg caggatggct cctgtcaccc cgtggctccc ggcctagttg 660
   gggccccaat gacccccggc gcaggtcgcg taatttgggt aaagtcatcg atacccttac 720
   atgcggcttc gccgacctca tggggtacat tccgctcgtc ggcgctccct tggggggcgc 780
   tgccagggcc ttggcgcatg gcgtccgggt tctggaggac ggcgtgaact atgcaacagg 840
   gaatctgccc ggttgctctt tctctatctt cctcttggct ctgctgtcct gtctaaccat 900
   cccagcttcc gcttatgaag tgcgcaacgt gtccggggtg taccatgtca cgaacgactg 960
   ctccaactcg agcattgtgt acgagacagg ggacatgatt atgcacaccc ctgggtgcgt 1020
   gccctgtgtt cgggagaaca actcctcccg ctgctgggca gcgctcactc ccacgctcgc 1080
   ggccaggaac gccagcgtcc ccaccacgac aatacggcgc cacgtcgatt tgctcgttgg 1140
   ggcggctgct ttctgctccg ctatgtacgt gggggatctc tgcggatctg ttttcctcgt 1200
   ctcccagttg ttcaccttct cgcctcgccg gcatgagaca gtgcaggact gcaattgttc 1260
   aatctatccc ggccacgtat caggtcaccg catggcttgg gatatgatga tgaactggtc 1320
   acctacaaca gccctactgg tatcgcagtt actccggatc ccacaagccg tcgtggacat 1380
   ggtggcgggg gcccactggg gagtcctggc gggccttgcc tactattcca tggcggggaa 1440
   ctgggctaag gttttgattg tgctgctact ctttgccggc gttgatgggg cgacctacgt 1500
   gacggggggg tcggaagcca gaggggcctc tggcttagca aacctctttt catttggggc 1560
   gtctcagaag atccagctca taaataccaa cggcagttgg cacatcaata gaactgccct 1620
   gaactgcaat gactccctcc acactgggtt tcttgccgcg ctattctaca cacacaaatt 1680
   caacgcgtcc ggatgtccag agcgcatggc cagctgccgc cccattgaag agttcgctca 1740
   ggggtatggt cccatcactt atgctgagcc ctccccctcg gaccagaggc cctattgctg 1800
   gcactacgcg cctcgaccgt gtggtatcat acccgcgtcg caggtgtgtg gtccagtgta 1860
   ctgcttcacc ccaagccctg ttgtggtggg gacgaccgat cgctccggtg cccccacgta 1920
   taattggggg gcgaatgaga cggacgtgct gtatctcaac aacacgcggc cgccgcaagg 1980
   caactggttc ggctgcacat ggatgaatgg caccgggttc accaagacgt gcgggggccc 2040
   cccgtgcaac atcggggggg gcggcaacaa caacaccttg acctgcccca cggactgttt 2100
   ccggaaacac cccgaggcca cctacaccaa atgtggttcg ggaccttggt tgacacctag 2160
   gtgcatggtc gactacccat acaggctctg gcactacccc tgcaccgtta actttaccat 2220
   ctttaaggtt aggatgtacg tgggaggtgt ggagcacagg ctcaacgccg catgcaattg 2280
   gacccgagga gagcgttgta acttagagga cagggataga tcagagctta gcccgctgct 2340
   gctgtcaaca acagagtggc aggtgctacc ttgttccttc accaccctac cggctctgtc 2400
   cactggtttg atccatctcc accagaacat cgtggacgtg caatacctgt acggtatagg 2460
   gtcggcggtt gtctcctatg caatcaaatg ggaatatgtc ttgttgctct tcctcctcct 2520
   ggcagacgcg cgcgtctgcg cctgcttgtg gatgatgctg ctgatagctc aagctgaggc 2580
   cgccttagag aacctggtgg tcctcaatgc ggcgtccctg gctggagcgc atggccttct 2640
   ctctttcctt gtgttcttct gtgccgcttg gtacatcaag ggcaggttga tccccggggc 2700
   ggcgtatgct ttttacggcg tatggccgct gctcctactc ctgctggcgt taccaccacg 2760
   agcatacgcc atggaccggg agatggctgc atcgtgcgga ggcgcggttt ttgtaggtct 2820
   ggcattcctg accttgtcac cacactataa ggcattcctc gccaagctcc tgtggtggtt 2880
   gtgctatctc ctgaccctgg gggaagccat gattcaggag tgggtaccac ccatgcaggt 2940
   gcgcggcggc cgcgatggca tcgcgtgggc cgtcactata ttctgcccgg gtgtggtgtt 3000
   tgacattacc aaatggcttt tggcgttgct tgggcctgct tacctcttaa gggccgcttt 3060
   gacacatgtg ccgtacttcg tcagagctca cgctctgata agggtatgcg ctttggtgaa 3120
   gcagctcgcg gggggtaggt atgttcaggt ggcgctattg gcccttggca ggtggactgg 3180
   cacctacatc tatgaccacc tcacacctat gtcggactgg gccgctagcg gcctgcgcga 3240
   cttagcggtc gccgtggaac ccatcatctt cagtccgatg gagaagaagg tcatcgtctg 3300
   gggagcggag acggctgcat gtggggacat tctacatgga cttcccgtgt ccgcccgact 3360
   cggccaggag atcctcctcg gcccagctga tggctacacc tccaaggggt ggaagctcct 3420
   tgctcccatc actgcttatg cccagcaaac acgaggcctc ctgggcgcca tagtggtgag 3480
   tatgacgggg cgtgacagga cagaacaggc cggggaagtc caaatcctgt ccacagtctc 3540
   tcagtccttc ctcggaacaa ccatctcggg ggttttgtgg actgtttacc acggagctgg 3600
   caacaagact ctagccggct tacggggtcc ggtcacgcag atgtactcga gtgctgaggg 3660
   ggacttggta ggctggccca gcccccctgg gaccaagtct ttggagccgt gcaagtgtgg 3720
   agccgtcgac ctatatctgg tcacgcggaa cgctgatgtc atcccggctc ggagacgcgg 3780
   ggacaagcgg ggagcattgc tctccccgag acccatttcg accttgaagg ggtcctcggg 3840
   ggggccggtg ctctgcccta ggggccacgt cgttgggctc ttccgagcag ctgtgtgctc 3900
   tcggggcgtg gccaaatcca tcgatttcat ccccgttgag acactcgacg ttgttacaag 3960
   gtctcccact ttcagtgaca acagcacgcc accggctgtg ccccagacct atcaggtcgg 4020
   gtacttgcat gctccaactg gcagtggaaa gagcaccaag gtccctgtcg cgtatgccgc 4080
   ccaggggtac aaagtactag tgcttaaccc ctcggtagct gccaccctgg ggtttggggc 4140
   gtacctatcc aaggcacatg gcatcaatcc caacattagg actggagtca ggaccgtgat 4200
   gaccggggag gccatcacgt actccacata tggcaaattt ctcgccgatg ggggctgcgc 4260
   tagcggcgcc tatgacatca tcatatgcga tgaatgccac gctgtggatg ctacctccat 4320
   tctcggcatc ggaacggtcc ttgatcaagc agagacagcc ggggtcagac taactgtgct 4380
   ggctacggcc acaccccccg ggtcagtgac aaccccccat cccgatatag aagaggtagg 4440
   cctcgggcgg gagggtgaga tccccttcta tgggagggcg attcccctat cctgcatcaa 4500
   gggagggaga cacctgattt tctgccactc aaagaaaaag tgtgacgagc tcgcggcggc 4560
   ccttcggggc atgggcttga atgccgtggc atactataga gggttggacg tctccataat 4620
   accagctcag ggagatgtgg tggtcgtcgc caccgacgcc ctcatgacgg ggtacactgg 4680
   agactttgac tccgtgatcg actgcaatgt agcggtcacc caagctgtcg acttcagcct 4740
   ggaccccacc ttcactataa ccacacagac tgtcccacaa gacgctgtct cacgcagtca 4800
   gcgccgcggg cgcacaggta gaggaagaca gggcacttat aggtatgttt ccactggtga 4860
   acgagcctca ggaatgtttg acagtgtagt gctttgtgag tgctacgacg caggggctgc 4920
   gtggtacgat ctcacaccag cggagaccac cgtcaggctt agagcgtatt tcaacacgcc 4980
   cggcctaccc gtgtgtcaag accatcttga attttgggag gcagttttca ccggcctcac 5040
   acacatagac gcccacttcc tctcccaaac aaagcaagcg ggggagaact tcgcgtacct 5100
   agtagcctac caagctacgg tgtgcgccag agccaaggcc cctcccccgt cctgggacgc 5160
   catgtggaag tgcctggccc gactcaagcc tacgcttgcg ggccccacac ctctcctgta 5220
   ccgtttgggc cctattacca atgaggtcac cctcacacac cctgggacga agtacatcgc 5280
   cacatgcatg caagctgacc ttgaggtcat gaccagcacg tgggtcctag ctggaggagt 5340
   cctggcagcc gtcgccgcat attgcctggc gactggatgc gtttccatca tcggccgctt 5400
   gcacgtcaac cagcgagtcg tcgttgcgcc ggataaggag gtcctgtatg aggcttttga 5460
   tgagatggag gaatgcgcct ctagggcggc tctcatcgaa gaggggcagc ggatagccga 5520
   gatgttgaag tccaagatcc aaggcttgct gcagcaggcc tctaagcagg cccaggacat 5580
   acaacccgct atgcaggctt catggcccaa agtggaacaa ttttgggcca gacacatgtg 5640
   gaacttcatt agcggcatcc aatacctcgc aggattgtca acactgccag ggaaccccgc 5700
   ggtggcttcc atgatggcat tcagtgccgc cctcaccagt ccgttgtcga ccagtaccac 5760
   catccttctc aacatcatgg gaggctggtt agcgtcccag atcgcaccac ccgcgggggc 5820
   caccggcttt gtcgtcagtg gcctggtggg ggctgccgtg ggcagcatag gcctgggtaa 5880
   ggtgctggtg gacatcctgg caggatatgg tgcgggcatt tcgggggccc tcgtcgcatt 5940
   caagatcatg tctggcgaga agccctctat ggaagatgtc atcaatctac tgcctgggat 6000
   cctgtctccg ggagccctgg tggtgggggt catctgcgcg gccattctgc gccgccacgt 6060
   gggaccgggg gagggcgcgg tccaatggat gaacaggctt attgcctttg cttccagagg 6120
   aaaccacgtc gcccctactc actacgtgac ggagtcggat gcgtcgcagc gtgtgaccca 6180
   actacttggc tctcttacta taaccagcct actcagaaga ctccacaatt ggataactga 6240
   ggactgcccc atcccatgct ccggatcctg gctccgcgac gtgtgggact gggtttgcac 6300
   catcttgaca gacttcaaaa attggctgac ctctaaattg ttccccaagc tgcccggcct 6360
   ccccttcatc tcttgtcaaa aggggtacaa gggtgtgtgg gccggcactg gcatcatgac 6420
   cacgcgctgc ccttgcggcg ccaacatctc tggcaatgtc cgcctgggct ctatgaggat 6480
   cacagggcct aaaacctgca tgaacacctg gcaggggacc tttcctatca attgctacac 6540
   ggagggccag tgcgcgccga aaccccccac gaactacaag accgccatct ggagggtggc 6600
   ggcctcggag tacgcggagg tgacgcagca tgggtcgtac tcctatgtaa caggactgac 6660
   cactgacaat ctgaaaattc cttgccaact accttctcca gagtttttct cctgggtgga 6720
   cggtgtgcag atccataggt ttgcacccac accaaagccg tttttccggg atgaggtctc 6780
   gttctgcgtt gggcttaatt cctatgctgt cgggtcccag cttccctgtg aacctgagcc 6840
   cgacgcagac gtattgaggt ccatgctaac agatccgccc cacatcacgg cggagactgc 6900
   ggcgcggcgc ttggcacggg gatcacctcc atctgaggcg agctcctcag tgagccagct 6960
   atcagcaccg tcgctgcggg ccacctgcac cacccacagc aacacctatg acgtggacat 7020
   ggtcgatgcc aacctgctca tggagggcgg tgtggctcag acagagcctg agtccagggt 7080
   gcccgttctg gactttctcg agccaatggc cgaggaagag agcgaccttg agccctcaat 7140
   accatcggag tgcatgctcc ccaggagcgg gtttccacgg gccttaccgg cttgggcacg 7200
   gcctgactac aacccgccgc tcgtggaatc gtggaggagg ccagattacc aaccgcccac 7260
   cgttgctggt tgtgctctcc ccccccccaa gaaggccccg acgcctcccc caaggagacg 7320
   ccggacagtg ggtctgagcg agagcaccat atcagaagcc ctccagcaac tggccatcaa 7380
   gacctttggc cagcccccct cgagcggtga tgcaggctcg tccacggggg cgggcgccgc 7440
   cgaatccggc ggtccgacgt cccctggtga gccggccccc tcagagacag gttccgcctc 7500
   ctctatgccc cccctcgagg gggagcctgg agatccggac ctggagtctg atcaggtaga 7560
   gcttcaacct cccccccagg gggggggggt agctcccggt tcgggctcgg ggtcttggtc 7620
   tacttgctcc gaggaggacg ataccaccgt gtgctgctcc atgtcatact cctggaccgg 7680
   ggctctaata actccctgta gccccgaaga ggaaaagttg ccaatcaacc ctttgagtaa 7740
   ctcgctgttg cgataccata acaaggtgta ctgtacaaca tcaaagagcg cctcacagag 7800
   ggctaaaaag gtaacttttg acaggacgca agtgctcgac gcccattatg actcagtctt 7860
   aaaggacatc aagctagcgg cttccaaggt cagcgcaagg ctcctcacct tggaggaggc 7920
   gtgccagttg actccacccc attctgcaag atccaagtat ggattcgggg ccaaggaggt 7980
   ccgcagcttg tccgggaggg ccgttaacca catcaagtcc gtgtggaagg acctcctgga 8040
   agacccacaa acaccaattc ccacaaccat catggccaaa aatgaggtgt tctgcgtgga 8100
   ccccgccaag gggggtaaga aaccagctcg cctcatcgtt taccctgacc tcggcgtccg 8160
   ggtctgcgag aaaatggccc tctatgacat tacacaaaag cttcctcagg cggtaatggg 8220
   agcttcctat ggcttccagt actcccctgc ccaacgggtg gagtatctct tgaaagcatg 8280
   ggcggaaaag aaggacccca tgggtttttc gtatgatacc cgatgcttcg actcaaccgt 8340
   cactgagaga gacatcagga ccgaggagtc catataccag gcctgctccc tgcccgagga 8400
   ggcccgcact gccatacact cgctgactga gagactttac gtaggagggc ccatgttcaa 8460
   cagcaagggt caaacctgcg gttacagacg ttgccgcgcc agcggggtgc taaccactag 8520
   catgggtaac accatcacat gctatgtgaa agccctagcg gcctgcaagg ctgcggggat 8580
   agttgcgccc acaatgctgg tatgcggcga tgacctagta gtcatctcag aaagccaggg 8640
   gactgaggag gacgagcgga acctgagagc cttcacggag gccatgacca ggtactctgc 8700
   ccctcctggt gatcccccca gaccggaata tgacctggag ctaataacat cctgttcctc 8760
   aaatgtgtct gtggcgttgg gcccgcgggg ccgccgcaga tactacctga ccagagaccc 8820
   aaccactcca ctcgcccggg ctgcctggga aacagttaga cactccccta tcaattcatg 8880
   gctgggaaac atcatccagt atgctccaac catatgggtt cgcatggtcc taatgacaca 8940
   cttcttctcc attctcatgg tccaagacac cctggaccag aacctcaact ttgagatgta 9000
   tggatcagta tactccgtga atcctttgga ccttccagcc ataattgaga ggttacacgg 9060
   gcttgacgcc ttttctatgc acacatactc tcaccacgaa ctgacgcggg tggcttcagc 9120
   cctcagaaaa cttggggcgc cacccctcag ggtgtggaag agtcgggctc gcgcagtcag 9180
   ggcgtccctc atctcccgtg gagggaaagc ggccgtttgc ggccgatatc tcttcaattg 9240
   ggcggtgaag accaagctca aactcactcc attgccggag gcgcgcctac tggacttatc 9300
   cagttggttc accgtcggcg ccggcggggg cgacattttt cacagcgtgt cgcgcgcccg 9360
   accccgctca ttactcttcg gcctactcct acttttcgta ggggtaggcc tcttcctact 9420
   ccccgctcgg tagagcggca cacactaggt acactccata gctaactgtt cctttttttt 9480
   tttttttttt tttttttttt tttttttttt tttttctttt tttttttttt ccctctttct 9540
   tcccttctca tcttattcta ctttctttct tggtggctcc atcttagccc tagtcacggc 9600
   tagctgtgaa aggtccgtga gccgcatgac tgcagagagt gccgtaactg gtctctctgc 9660
   agatcatgt 9669

<210> 4
<211> 21
<212> DNA
<213> Artificial

<220>
<223> synthetic oligonucleotide Mod87

<400> 4
gggggggcga cgatcgtcag g 21

<210> 5
<211> 21
<212> DNA
<213> Artificial

<220>
<223> synthetic oligonucleotide Mod87s

<220>
<223> (1)..(7),(20) phosphorothioated guanine

<400> 5
nnnnnnncga cgatcgtcan g 21

<210> 6
<211> 28
<212> DNA

<213>  Artificial

<220>
<223>  primer J6E2Fc-s

<400>  6
cacaagcttc gcacccatac tgttgggg                                          28


<210>  7
<211>  30
<212>  DNA
<213>  Artificial

<220>
<223>  primer J6E2Fc-as

<400>  7
acaggatccc atcggacgat gtattttgtg                                        30


<210>  8
<211>  32
<212>  DNA
<213>  Artificial

<220>
<223>  primer J6E2dTM-as

<400>  8
gctctagatt accatcggac gatgtatttt gt                                     32


<210>  9
<211>  30
<212>  DNA
<213>  Artificial

<220>
<223>  primer J6E1F-s

<400>  9

cacaagcttg ccgaagtgaa gaacatcagt 30

<210> 10
<211> 32
<212> DNA
<213> Artificial

<220>
<223> primer J6E1F-as

<400> 10
tcgggatccc aatgagcccc gctaatgatg tc 32